# EUROPEAN PATENT SPECIFICATION

(11) **EP 1 241 182 B1**
(45) Date of publication and mention of the grant of the patent: **19.10.2011**
(21) Application number: 02005749.3
(22) Date of filing: 13.03.2002
(51) Int. Cl.: C07K 14/705, C12N 15/29, G01N 33/50, C12N 1/19

(54) **Analysis of agonist-activity and antagonist-activity to cytokinin receptor**
Analyse der Agonist- und Antagonistaktivitäten gegenüber einem Cytokininrezeptor
Analyse d'activité agoniste et antagoniste contre le récepteur de cytokinine

(30) Priority: 15.03.2001 JP 2001073812; 29.06.2001 JP 2001198639; 29.06.2001 JP 2001198640
(43) Date of publication of application: 18.09.2002
(60) Divisional application: 09011776.3
(73) Proprietor: Sumitomo Chemical Company, Limited, Tokyo 104-8260 (JP)
(72) Inventor: Kakimoto, Tatsuo, Toyonaka-shi, Osaka (JP); Higuchi, Masayuki, Okayama-shi, Okayama (JP); Inoue, Tsutomu, Osaka-shi, Osaka (JP)
(74) Representative: Vossius & Partner

(56) References cited:
- EP-A- 0 892 063
- WO-A-99/36508
- US-A- 6 077 682
- INOUE TSUTOMU ET AL: "Identification of CRE1 as a cytokinin receptor from Arabidopsis." NATURE (LONDON), vol. 409, no. 6823, 2001, pages 1060-1063, XP001061408 ISSN: 0028-0836 -& DATABASE EMBL [Online] AB049934; XP002199637
- MAHONEN ARI PEKKA ET AL: "A novel two-component hybrid molecule regulates vascular morphogenesis of the Arabidopsis root." GENES & DEVELOPMENT, vol. 14, no. 23, 1 December 2000 (2000-12-01), pages 2938-2943, XP001079006 ISSN: 0890-9369
- KAKIMOTO T: "CKI1, a histidine kinase homolog implicated in cytokinin signal transduction" SCIENCE, AMERICAN ASSOCIATION FOR THE ADVANCEMENT OF SCIENCE,, US, vol. 274, 8 November 1996 (1996-11-08), pages 982-985, XP002102276 ISSN: 0036-8075
- UEGUCHI C ET AL: "NOVEL FAMILY OF SENSOR HISTIDINE KINASE GENES IN ARABIDOPSIS THALIANA" PLANT AND CELL PHYSIOLOGY, JAPANESE SOCIETY OF PLANT PHYSIOLOGISTS, XX, vol. 2, no. 42, February 2001 (2001-02), pages 231-235, XP001079007 ISSN: 0032-0781 -& DATABASE EMBL [Online] retrieved from EMBL Database accession no. Q9C5U1 XP002212289
- DATABASE EBI [Online] Database accession no. Q9C5U2 XP002212290
- KAKIMOTO T: "CYTOKININ SIGNALING" CURRENT OPINION IN PLANT BIOLOGY, QUADRANT SUBSCRIPTION SERVICES, GB, vol. 5, no. 1, 1998, pages 399-403, XP001079011 ISSN: 1369-5266
- ESTELLE M: "CYTOKININ ACTION: TWO RECEPTORS BETTER THAN ONE?" CURRENT BIOLOGY, CURRENT SCIENCE,, GB, vol. 16, no. 8, 1998, pages R539-R541, XP001071265 ISSN: 0960-9822
- URAO TAKESHI ET AL: "A transmembrane hybrid-type histidine kinase in Arabidopsis functions as an osmosensor." PLANT CELL, vol. 11, no. 9, 1999, pages 1743-1754, XP002212287 ISSN: 1040-4651
- KULAEVA O N ET AL: "A new family of cytokinin receptors from Cereales." FEBS LETTERS, vol. 423, no. 2, 20 February 1998 (1998-02-20), pages 239-242, XP002212288 ISSN: 0014-5793

## Description

### BACKGROUND OF THE INVENTION

### FIELD OF THE INVENTION

The present invention relates to a method for analyzing agonist-activity and antagonist-activity of an examinee to cytokinin receptor.

### DESCRIPTION OF THE RELATED ART

Cytokinins are plant hormones relevant to cell division and differentiation of higher plants and are well-known as very important physiologically active substances having functions of inducing division of cells of higher plants, differentiating the callus and pith to the stems and leaves, ethiolating and defoliating leaves, preventing the falling of fruit, breaking the dominance of the terminal bud and the like [Cytokinins: Chemistry, Activity, and Function, CRC press (1994)]. The substances having cytokinins-like activities are usable as plant growth regulators, for example, fruit-falling preventive agents for fruits such as apple, orange, and the like, plant-falling preventive agents for rice plants, barley, wheat and the like by regulating the height of the plants, and sweetness increasing agents for fruits after harvest.

As a method for finding substances having such cytokinins-like activities, conventionally employed is a method for observing and evaluating the physiological changes by directly spraying examinee substances to plant.

The aforementioned method has problems that it requires to prepare the examinee substances in amounts sufficient to directly spray them to plant and also requires immensely long time to observe and evaluate the growth of the plant and the physiological changes of the plant after spraying of the examinee substances. Therefore, it has been desired that a variety of methods be developed for quickly finding substances having cytokinins-like activities with small amounts of examinee substances.

### SUMMARY OF THE INVENTION

Inventors of the present invention have intensively investigated in such situations and first found protein functioning as a cytokinin receptor and subsequently found it possible to analyze the agonist-activity and the antagonist-activity of examinee substances to a cytokinin receptor by using the cytokinin receptor ("the analysis method") and found it also possible to quickly search substances having cytokinins-like activities even in a small amount of examinee substances by employing the analysis method and consequently reached the present invention.
The present invention provides:
1. A method for analyzing antagonist-activity to a cytokinin receptor, which comprises
   (1) culturing a transformed cell into which a DNA encoding a cytokinin receptor is introduced in a liquid culture medium containing an examinee substance and a substance having agonist-activity to the cytokinin receptor; and
   (2) measuring an existence or quantity of intracellular signal transduction from the cytokinin receptor expressed in the transformed cell,
   wherein growth of the transformed cell is controlled by intracellular signal transduction from the cytokinin receptor, and wherein the existence or the quantity of the intracellular signal transduction is determined by measuring growth of the transformed cell, and
   wherein the substance having agonist-activity is selected from the group consisting of: trans-zaetin, cis-zeatin, benzyl adenine and thidiazuron.
2. A method for analyzing agonist-activity to a cytokinin receptor, which comprises
   (1) culturing a transformed cell into which a DNA-encoding a cytokinin receptor is introduced in a liquid culture medium containing an examinee substance; and
   (2) measuring the existence or the quantity of intracellular signal transduction from the cytokinin receptor expressed in the transformed cell,
   wherein growth of the transformed cell is controlled by intracellular signal transduction from the cytokinin receptor, and wherein the existence or the quantity of the intracellular signal transduction is determined by measuring growth of the transformed cell.
3. The method of 1 or 2, wherein the transformed cell is a transformed cell generated by introducing DNA encoding the cytokinin receptor into a host cell so improved as to have histidine kinase activity lower than the intrinsic histidine kinase activity of the host cell.
4. The method of any one of 1 to 3, wherein the transformed cell is a transformed cell generated by introducing DNA encoding the cytokinin receptor into a host cell so improved as to have histidine kinase activity lower than the intrinsic histidine kinase activity of the host cell by deleting one or more of histidine kinase.
5. The method of 1 or 2, wherein the transformed cell is a transformed cell generated by introducing DNA encoding the cytokinin receptor into a host cell having no cytokinin receptor.
6. The method of any one of 1 to 5, wherein the transformed cell is yeast.
7. The method of 6, wherein the transformed cell is budding yeast.
8. A method for detecting antagonist-activity to a cytokinin receptor, which comprises evaluating the antagonist-activity of two or more different examinee substances to the cytokinin receptor based on the difference obtained by comparison of the existence or the quantity of the intracellular signal transduction in a section where the examinee substances are independently used and measured by the analysis method of 1.
9. The method of 8, wherein at least one substance among the two or more different examinee substances is a substance having no antagonist-activity to the cytokinin receptor.
10. A method for detecting agonist-activity to a cytokinin receptor, which comprises evaluating the agonist-activity of two or more different examinee substances to the cytokinin receptor based on the difference obtained by comparison of the existence or the quantity of the intracellular signal transduction in a section where the examinee substances are independently used and measured by the analysis method of 2.
11. The method of 10, wherein at least one substance among the two or more different examinee substances is a substance having no agonist-activity to the cytokinin receptor.

Further scope of applicability of the methods described heroin will become apparent from the detailed description given hereinafter. However, it should be understood that the detailed description and specific examples, while indicating preferred embodiments of the invention, are given by way of illustration only

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

### DETAILED DESCRIPTION OF THE PREFERRED EMBODIMENTS

A cytokine receptor is a protein having functions of controlling the propagation and differentiation of cells of higher plants based on the intracellular signal transduction mechanism so-called Two-Component regulatory system (or Histidine to Aspartic acidartic acid phosphorelay system) while being specifically bonded with cytokinins such as purine type cytokinins, e.g. kinetinee, zeatinee, and urea type cytokinins, e.g. N-phenyl-N'-(4-pyridyl)urea. The cytokinin receptor to be used in the present invention belongs to the histidine kinase family and is protein composed of extracellular regions, transmembrane regions, histidine kinase regions (regions having histidine kinase activity in the cell and holding Histidine residue to be an active site), and receiver regions (regions having a reception part for phosphate group transfer and holding Aspartic acid residue to be an active site).

Practical examples of the cytokinin receptor are cytokinin receptors having the amino acid sequence represented by SEQ ID No: 2; cytokinin receptors having the amino acid sequence represented by SEQ ID No: 4; cytokinin receptors having the amino acid sequence represented by SEQ ID No: 6; cytokinin receptors having the amino acid sequence represented by SEQ ID No: 2 wherein one or a plurality of amino acids are deleted, substituted, or added; cytokinin receptors having the amino acid sequence represented by SEQ ID No: 4 wherein one or a plurality of amino acids are deleted, substituted, or added; cytokinin receptors having the amino acid sequence represented by SEQ ID No: 6 wherein one or a plurality of amino acids are deleted, substituted, or added; cytokinin receptors having the amino acid sequence coded with DNA to be hybridized in stringent conditions with DNA having the nucleotide sequence coding the amino acid sequences represented by SEQ ID No: 2; cytokinin receptors having the amino acid sequence coded with DNA to be hybridized in stringent conditions with DNA having the nucleotide sequence coding the amino acid sequences represented by SEQ ID No: 4; cytokinin receptors having the amino acid sequence coded with DNA to be hybridized in stringent conditions with DNA having the nucleotide sequence coding the amino acid sequences represented by SEQ ID No: 6; cytokinin receptors of partially transmembrane region-deleted type which will be described later; or chimera-type cytokinin receptors which will be described later. Incidentally, the phrase, "a plurality of amino acids", means more particularly about 2 to 20 amino acids and for example, 2 to 10 amino acids and 2 to 5 amino acids may be exemplified. Also, the phrase, "the amino acid sequences ... wherein one or a plurality of amino acids are deleted, substituted, or added", means as examples those having the amino acid sequences of which 80% or higher, preferably 90% or higher, and more preferably 95% or higher are identical with the sequences of the amino acids before the deletion, substitution or addition of amino acids (i.e. amino acid sequence identification of 80% or higher, particularly 90% or higher, and more preferably 95% or higher).

Those phrases, "deletion, substitution or addition of amino acids" or "which 80% or higher ... are identical" of course include the proteins provided by the intracellular processing to which proteins having the amino acid sequences represented by SEQ ID Nos: 2, 4, and 6 are subjected, and the natural variations caused by differences in type of organisms from which the proteins are derived, differences in individual bodies, or differences in tissues.

The phrase, "sequence identification", in the present invention means the identification and homology between two DNA sequences and between two protein sequences. The sequence identification may be determined by comparing two sequences aligned in the optimum states in a region of the sequences of the comparison objects. The DNA or proteins, the comparison objects, may have addition or deletion (e.g. gap) in the optimum alignment of two sequences. Regarding such sequence identification, computation may be performed using, for example, Vector NTI by producing alignment by utilizing Clusta 1W algorithm [Nucleic Acid Res., 22 (22): 4673-4680 (1994)]. Incidentally, the sequence identification may be measured by a sequence analysis soft, practically Vector NTI, GENETYX-MAC and analysis tools provided in public database. The aforementioned public database may be, in general, accessible in, for example, home address; http://www.ddbj.nig.ac.jp.

Regarding the phrase, "be hybridized in stringent conditions", the term, hybridization, in this case may be performed, for example, according to a common method described in Molecular Cloning 2nd edition, written by Sambrook J., Frisch E. F., Maniatis T., issued by Cold Spring Harbor Laboratory press. Further, the phrase, "in stringent conditions", means, for example, that a hybrid is formed in a solution of 6 × SSC (a solution containing 1.5 M NaCl and 0.15 M trisodium citrate is defined as 10 × SSC) at 65 °Cand then washed with 1 × SSC at a room temperature. The salt concentration in the washing step may be selected, for example, from the condition of 1 × SSC at a room temperature (a low stringent condition) to 0.1 × SSC at a room temperature (a high stringent condition). The temperature in the washing step may be selected, for example, from a room temperature (a low stringent condition) to 68°C (a high stringent condition). Further, both of the salt concentration and the temperature may be changed. (Production of a transformed cell into which DNA coding a cytokinin receptor is introduced)

A transformed cell into which DNA coding a cytokinin receptor is introduced may be obtained by introducing and expressing a DNA coding one of the above cytokinin receptors. For example, DNA having nucleotide sequence coding the amino acid sequence of the cytokinin receptor can be introduced into a host cell in the following manner. Hereinafter, one example of the method of the production of a transformed cell will be described.

### (1) Preparation of cDNA

At first, the total RNA is prepared from plants such as higher plants according to the method described in Molecular Cloning 2nd edition written by J., Sambrook, E., F., Frishch, T., Maniastis.

Concretely, for example, after a part of tissues are sampled from a higher plant, the tissues are frozen in liquefied nitrogen and successively physically milled using a mortar and pestle or the like. The total RNA may then be obtained by methods in which (a) the resulting milled product is mixed with a solution containing guanidine hydrochloride with phenol or SDS with phenol to obtain the total RNA or (b) the resulting milled product is mixed with a solution containing guanidine thiocyanate and further with CsCl and then subjected to centrifugal separation to obtain the total RNA. Examples of the higher plant include a monocotyledonous plant, e.g. rice, corn, barley, wheat and the like and a dicotyledonous plant, e.g. tobacco, soybean, Arabidopsis, and the like. For the aforementioned process, e.g., commercialized kits such as ISOGEN (produced by Nippon Gene Co.), or RNeasy Total RNA Purification Kit (produced by QIAGEN Co.), may be employed.

Next, mRNA is prepared from the total RNA. For example, the preparation may be carried out by a method utilizing the hybridization of oligo-dT chains bonded with cellulose or latex and poly-A chains of mRNA. For the operation, for example, commercialized kits such as mRNA Purification Kit (produced by Amersham Pharmacia Co.), Oligotex TM-dT30 (super) (produced by Takara Shuzo Co. Ltd.) may be employed.

Further, cDNA is produced using the mRNA (mRNA having poly-A chains) may be prepared in the following manner. For example, oligo-dT chains or random primers are annealed with mRNA and then reacted with reverse transcriptase to produce cDNA. Further, the cDNA is reacted with, for example, RNaseH, DNA polymerase I to produce double chain cDNA. For the operation, for example, the following commercialized kits may be employed: SMARTTM PCR cDNA Synthesis Kit (produced by Clontech Co.), cDNA Synthesis Kit (produced by Takara Shuzo Co. Ltd.), cDNA Synthesis Kit (produced by Amersham Pharmacia Co.), or ZAP-cDNA Synthesis Kit (produced by Stratagene Co.).

### (2) Cloning

The DNA coding the cytokinin receptor may be obtained by a polymerase chain reaction (hereinafter referred as to PCR) from the produced cDNA using, for example, DNA having partial nucleotide sequence of the nucleotide sequence of SEQ ID No: 1, 3 or 5 as a primer or by a hybridization method using DNA having partial nucleotide sequence of the nucleotide sequence of SEQ ID No: 1, 3 or 5 as a probe.

In the case of employing PCR, DNA usable as a primer set is those planned and synthesized based on the nucleotide sequences of about 20 bp to 40 bp, for example, the nucleotide sequences selected respectively from 5'-non-translation regions and 3'-non-translation regions of the nucleotide sequence represented by SEQ ID No: 1, 3, or 5. Examples of the primer set are sets of DNA of nucleotide sequence represented by SEQ ID No: 9 and DNA of nucleotide sequence represented by SEQ ID No: 10. The PCR solution to be used may be prepared by adding reaction solutions instructed by the kit to cDNA 250 ng. The conditions of the PCR may properly be changed depending on the primer set to be used and, for example, concrete conditions include as follows: keeping at 94°C for 2 minutes, at about 8°C for 3 minutes, and further repeating 40 cycles each of which comprises steps of keeping at 94°C for 30 seconds, at 55 °C for 30 seconds, and at 72°C for 4 minutes; and repeating 5 to 10 cycles each of which comprises steps of keeping at 94°C for 5 seconds and at 72°C for 4 minutes and further repeating about 20 to 40 cycles each of which comprises steps of keeping at 94°C for 5 seconds and at 70°C for 4 minutes. For the operation, the following commercialized kits may be, for example, employed: Takara Heraculase^{™} (produced by Takara Shuzo Co., Ltd.), DNA polymerase contained in Advantage cDNA PCR Kit (Clontech Co.), TAKARA Ex Taq (Takara Shuzo Co., Ltd.), or PLATINUMTM PCR SUPER Mix (Lifetech Oriental Co.).

In the case of employing hybridization, cloning may be carried out according to a method described in, for example, "Cloning and Sequence", Experimental Manual of Plant Biotechnology (edited by Watanabe and Sugiura, Noson Bunka Publisher, 1989).

The probe to be used may be obtained by synthesizing DNA (with the chain length of about 200 nucleotides to 500 nucleotides) having partial nucleotide sequences of the nucleotide sequence represented by SEQ ID No: 1, 3 or 5 and labelling the DNA with radioisotope markers or fluorescent markers according to the known methods using, for example, Random Primed DNA Labelling Kit (Boehringer Co.), Random Primer DNA Labelling Kit Ver. 2 (Takara Shuzo Co., Ltd.), ECL Direct Nucleic Acid Labelling and Detection System (Amersham Pharmacia Co.), or Megaprime DNA-labelling system (Amersham Pharmacia Co.).

Examples of the hybridization conditions include stringent conditions and the following conditions may be exemplified: keeping at 65°C in the presence of 6 × SSC (0.9 M NaCl and 0.09 M trisodium citrate), 5 × Denhard's solution [0.1% (w/v) ficoll 400, 0.1% (w/v) polyvinylpyrrolidone, 0.1% BSA], 0.5% (w/v) SDS and 100 µg/ml degenerated salmon sperm DNA or in DIG EASY Hyb solution (Boeringer-Mannheim Co.) containing 100 µ g/ml of degenerated salmon sperm DNA, successively keeping at a room temperature for 15 minutes two times in the presence of 1 × SSC (0.15 M NaCl and 0.015 M trisodium citrate) and 0.5% (w/v) SDS, and further keeping at 68°C for 30 minutes in the presence of 0.1 × SSC (0.015 M NaCl and 0.0015 M trisodium citrate) and 0.5% SDS.

In order to obtain DNA coding the cytokinin receptor of Arabidopsis, PCR can be carried out employing TAKARA LA taq^{™} (Takara Shuzo Co., Ltd.) and using a solution containing cDNA library phage of Arabidopsis (about 1,000,000 pfu) as a template and DNA having the nucleotide sequence represented by SEQ ID No: 11 and DNA having the nucleotide sequence represented by SEQ ID No: 12 as a primer set to amplify and obtain DNA to be a probe. The PCR solution to be used may be prepared by adding to 250 ng of cDNA library, the reaction solutions instructed by the kit.

The PCR conditions may be as follows: keeping at 94 °C for 2 minutes, at 8 °C for 3 minutes, and further repeating 40 cycles each of which comprising keeping at 94 °C for 30 seconds, at 55 °C for 30 seconds, and at 68 °C for 5 minutes.

Using the amplified and obtained DNA as a template, a ³²P-labeled probe may be produced employing Megaprime DNA-labelling system kit (Amersham Pharmacia Co.) and using the reaction solutions instructed by the kit. Using the probe obtained in such a manner, colony hybridization is carried out by a conventional method, practically the hybridization is carried out keeping at 65 °C in the presence of 6 × SSC (0.9 M NaCl and 0.09 M trisodium citrate), 5 × Denhard's solution [0.1% (w/v) ficoll 400, 0.1% (w/v) polyvinylpyrrolidone, 0.1% BSA], 0.5% (w/v) SDS and 100 µg/ml degenerated salmon sperm DNA or in the DIG EASY Hyb solution (Boeringer-Mannheim Co.) containing 100 µg/ml of degenerated salmon sperm DNA, successively keeping at a room temperature for 15 minutes two times in the presence of 1 × SSC (0.15 M NaCl and 0.015 M trisodium citrate) and 0.5% (w/v) SDS, and further keeping at 68 °C for 30 minutes in the presence of 0.1 × SSC (0.015 M NaCl and 0.0015 M sodium citrate) and 0.5% SDS to obtain clone hybridized with the probe.

Further, DNA coding the cytokinin receptor may be prepared based on, for example, the nucleotide sequence represented by SEQ ID No: 1, 3 or 5 by chemical synthesis of polynucleotides according to a common method such as phosphite-triester method (Hunkapiller, M. et al., Nature, 310, 105, 1984).

The DNA coding the cytokinin receptor obtained in such a manner may be cloned in a vector by a common method described in, e.g., "Molecular Cloning: A Laboratory Manual 2nd edition" (1989), Cold Spring Harbor Laboratory Press; "Current Protocols In Molecular Biology" (1987), John Wiley & Sons, Inc. ISBNO-471-50338-X. The vector to be used may be, for example, pBlue Script II vector (produced by Stratagene Co.), pUC 18/19 vector (produced by Takara Shuzo Co., Ltd.), or TA cloning vector (produced by Invitrogen Co.).

Incidentally, the nucleotide sequence of cloned DNA may be confirmed by, the Maxam Gilbert method (described in, for example, Maxam, A., M & W. Gilbert, Proc. Natl. Acad. Sci. USA, 74, 560, 1977), the Sanger method (described in, for example, Sanger, F. & A.R. Coulson, J. Mol. Biol., 94, 441, 1975, or Sanger, F. & Nicklen and A.R. Coulson., Proc. Natl. Acd. Sci. USA, 74, 5463, 1977). For the process, the following commercialized kits may be, for example, employed: Thermo Sequenase II dye terminator cycle sequencing kit (produced by Amersham Pharmacia Co.), or Dye Terminator Cycle Sequencing FS Ready Reaction Kit (produced by PE Biosystems Japan Co.). (3) Construction of expression vector

A expression vector of DNA coding the cytokinin receptor may be constructed according to a common method described in, for example, Molecular Cloning 2nd edition written by J., Sambrook, E., F., Frisch, & T., Maniastis, published by Cold Spring Harbor Laboratory Press.

Usable are vectors to be used in host cells to be transformed, for example, independently replicating vectors which contain genetic information possible to be duplicated in the host cells and further are possible to be isolated from the host cells and purified and may have detectable marker. More practically, in the case of using bacteria such as E. coli as the host cells, for example, Plasmid pUC 119 (produced by Takara Shuzo Co., Ltd.), or Phagemid pBluescript II (Stratagene Co.) may be used. In the case of using yeast as the host cells, for example, Plasmid pACT2 (Clontech Co.) may be used. In the case of using plant cells as the host cells, for example, DNA coding the cytokinin receptor may be integrated with Plasmid pBI221 (Clontech Co.) to construct the vectors.

A expression vector possible to express DNA coding the cytokinin receptor in a host cell may be constructed by integrating a promoter with the aforementioned vectors in the upstream of the DNA coding the cytokinin receptor in a binding manner of enabling to function in the host cell. In this case, the phrase, "in a binding manner of enabling to function", means that the promoter and the DNA coding the cytokinin receptor are bonded as to express the DNA coding the cytokinin receptor in the host cell under the control of the promoter. Usable as the promoter possible to function in the host cell in the case of using E. coli as the host cell are, for example, a promoter (*lacP*) of lactose operon of E. coli, a promoter (*trpP*) of triptophan operon, a promoter (*argP*) of arginine operon, a promoter (*gslP*) of galactose operon, a tac-promoter, T7-promoter, T3-promoter,or λ-phage promoter, (λ -pL, λ -pR). In the case of using yeast as the host cell, it may be prepared by a conventional genetic engineering method [described in Method in Enzymology 101 part (p.192-201) by Ammerer, et. al.] from *ADH1* promoter (the *ADH1* promoter is available from the yeast expression vector pAAH5 which contains the *ADH1* promoter and its terminator and which may be obtained from Washington Research Foundation). The *ADH1* promoter is included in US patent application number 299,733 of Washington Research Foundation and in the case that it is used for industrial and commercial purposes in USA, it is required to obtain permission from the patent holder. In the case of using a plant cell as the host cell, usable examples are a nopaline synthesis enzyme gene (*NOS*) promoter, an octopine synthesis enzyme gene (*OCT*) promoter, a cauliflower mosaic virus (CaMV)-derived 19S promoter, or a CaMv-derived 35S promoter.

Further, in the case of integrating DNA coding the cytokinin receptor into a vector already having a promoter possible to function in a host cell, DNA coding the cytokinin receptor is inserted in the downstream of the promoter and the DNA coding the cytokinin receptor is inserted in a manner of enabling to function. For example, the aforementioned plasmid pACT 2 for yeast comprises the *ADH1* promoter and therefore, an expression vector possible to express the DNA coding the cytokinin receptor in yeast, such as CG1945 (Clontech Co.) may be constructed by inserting the DNA coding the cytokinin receptor in the downstream of the *ADH1* promoter of the plasmid pACT2.

### (4) Production of transformed cell

A transformed cell to be used in the present invention may be produced by introducing the constructed expression vector into a host cell by a conventional method. As the host cell to be used for production of the transformed cell, examples are bacteria, yeast, or plant cell. As bacteria, examples are bacteria belonging to E. coli, Serratia, Bacillus, Brevibacterium, Corynebacterium, or Microbacterium. As yeast, examples are budding yeast and fission yeast. More particularly, examples are yeast belonging to Saccharomyces, or Schizosaccharomyces. As plant cell, examples are BY-2 strain, which is a cultured cell of tobacco, and BMS strain, which is a cultured cell of corn (Black Mexican Sweet).

As the method for introducing the expression vector into the aforementioned host cell, conventional introduction methods are employed according to the host cell to be transformed. For example, in the case of using bacteria as the host cell, the aforementioned expression vector may be introduced into a host cell by employing a conventional introduction method such as a calcium chloride method and an electroporation method described in, "Molecular Cloning" (by J. Sambrook, et. al Cold Spring Harbor 1989). In the case of using yeast as a host cell, the aforementioned expression vector may be introduced into the host cell by employing Yeast transformation kit (produced by Clontech Co.) based on a lithium method. Further, in the case of using a plant cell as a host cell, the aforementioned expression vector may be introduced into the host cell by a conventional introduction method, for example, Agrobacterium infection method (Japanese Examined Patent Application No. 2 - 58917, Japanese Laid-Open Patent Application No. 60- 70080), an electroporation method into a protoplast (Japanese Laid-Open Patent Application No. 60- 251887, Japanese Laid-Open Patent Application No. 5- 68575), a particle gun method (Japanese Laid-Open Patent Application No. 6- 508316, Japanese Laid-Open Patent Application No. 63 - 258525).
(Transformed cell in which a cytokinin receptor of partially transmembrane region-deleted type, i.e. a cytokinin receptor of a transmembrane quantity variation type, is expressed)

A cytokinin receptor to be used in the present invention includes a cytokinin receptor wherein said cytokinin receptor has at least one transmembrane region but less than that in its natural form (commonly 2 to 4 transmembrane region) (incidentally, in the present invention, such cytokinin receptors are sometimes referred as to cytokinin receptors of partially transmembrane region-deleted type.) In this case, the phrase, "its natural form" means cytokinin receptors having an amino acid sequence most frequently existing among organisms having the similar nomenclature and generally called also as wild-type cytokinin receptor.

Such cytokinin receptors of partially transmembrane regions-deleted type are cytokinin receptors whose transmembrane region structure may be determined by employing structure assumption software available in http://www.ch.embnet.org/software/TMPRED_form.html and whose transmembrane regions are partially deleted, for example, in 1 to 2 sites and are less in number than the number of the transmembrane regions of the natural type cytokinin receptors (i.e. natural form).

More particularly, examples of such cytokinin receptors include a cytokinin receptor having the amino acid sequence from amino acid number 196 to 1176 among the amino acid sequence represented by SEQ ID No: 2 (2 transmembrane regions); a cytokinin receptor having the amino acid sequence from amino acid number 50 to 1176 among the amino acid sequence represented by SEQ ID No: 2 (3 transmembrane regions); a cytokinin receptor having the amino acid sequence from amino acid number 32 to 1036 among the amino acid sequence represented by SEQ ID No: 4 (2 transmembrane regions); a cytokinin receptor having the amino acid sequence derived from the amino acid sequences of these cytokinin receptors wherein one or a plurality of amino acids are deleted, substituted or added, for example, cytokinin receptors having the amino acid sequence derived from the amino acid sequences in which one methionine is added to the amino-terminal.

The DNA coding the cytokinin receptor may be constructed as to hold transmembrane regions in a less number than the number of the transmembrane regions of the natural type cytokinin receptors by partially deleting the transmembrane regions by a conventional genetic engineering technique.

Production of the transformed cell in which the DNA coding the cytokinin receptor is introduced may be carried out according to the aforementioned method, "Production of transformed cell in which the DNA coding a cytokinin receptor is introduced".

### (Transformed cell to express chimera-type cytokinin receptor)

A cytokinin receptor to be used in the present invention also includes a chimera-type cytokinin receptor comprising an extracellular region of the cytokinin receptor, transmembrane regions of the cytokinin receptor, a histidine kinase region of the cytokinin receptor, and a receiver region for a histidine kinase. The chimera-type cytokinin receptor has the extracellular region, the transmembrane regions, and the histidine kinase region homogeneous to one another and the receiver region for the histidine kinase heterogeneous to these regions. For example, the chimera-type cytokinin receptor may have said extracellular region, transmembrane regions, and histidine kinase region derived from a cytokinin receptor selected from CRE1, AHK2, and AHK3, and have the receiver region derived from the osmosensor SLN1 in budding yeast.

In the above, histidine kinase regions of the cytokinin receptor may include the region present C-terminally downstream from the particular transmembrane region which is the most C-terminally downstream transmembrane region of the cytokinin receptor. Said histidine kinase region typically has five conservative motifs which are common to generic histidine kinases as described in Annual Review of Genetics 23:311-336 (1989), Microbiological Reviews 53(4):450-490(1989), or Science 262:539-544(1993). Examples of the region includes a region having the amino acid sequence from amino acid number 587 to 844 among the amino acid sequence represented by SEQ ID No: 2 in a case of AHK2, a region having the amino acid sequence from amino acid number 450 to 700 among the amino acid sequence represented by SEQ ID No: 4 in a case of AHK3 and a region having the amino acid sequence from amino acid number 449 to 714 among the amino acid sequence represented by SEQ ID No: 6 in a case of CRE1.

Receiver regions of the cytokinin receptor may include the region existing between the histidine kinase region and the C-terminal end of the cytokinin receptor. Said region having three conservative motifs which are common to generic histidine kinases as described in, e.g., Annual Review of Genetics 23:311-336 (1989), Science 262:539-544(1993).
Examples of the region includes a region having the amino acid sequence from amino acid number 891 to 1163 among the amino acid sequence represented by SEQ ID No: 2 in a case of AHK2, a region having the amino acid sequence from amino acid number 746 to 1018 among the amino acid sequence represented by SEQ ID No: 4 in a case of AHK3 and a region having the amino acid sequence from amino acid number 763 to 1038 among the amino acid sequence represented by SEQ ID No: 6 in a case of CRE 1.

In addition, a sensor region for cytokinin means, for example, a region which is a part of any of the extracellular regions of the cytokinin receptor, wherein said sensor region is present between a transmembrane region next to the histidine kinase region and a transmembrane region secondary close to the histidine kinase region. Said sensor region typically has 50% or more identification and homology between three cytokinin receptors of AHK2, AHK3 and CRE1 as described in Plant and Cell Physiology 42(2):231-235(2001). Examples of the region includes a region having the amino acid sequence from amino acid number 259 to 536 among the amino acid sequence represented by SEQ ID No: 2 in a case of AHK2, a region having the amino acid sequence from amino acid number 120 to 399 among the amino acid sequence represented by SEQ ID No: 4 in a case of AHK3 and a region having the amino acid sequence from amino acid number 132 to 398 among the amino acid sequence represented by SEQ ID No: 6 in a case of CRE1.

The histidine kinase protein has the following sequence in common in plants, e.g. higher plants, and microorganism. That is, histidine kinase protein is composed of an extracellular region, transmembrane region (generally about 2 to 4), histidine kinase region having histidine kinase activity and holding Histidine residue to be an active site, and receiver region having a reception part for a phosphoryl group transfer and holding Aspartic acid residue to be an active site. In the chimera-type cytokinin receptor, it is preferable that the extracellular region, the transmembrane regions, and the histidine kinase region are all derived from the identical type of cytokinin receptor, whereas the receiver regions are derived differently from type of the former cytokinin receptor.

It is sufficient for the receiver region of the chimera-type cytokinin receptor to have a function of receiving signals transmitted from the histidine kinase region and transmitting them to the next step and any of receiver region may be usable as long as they can complement or improve the intrinsic functions of the receiver region of a histidine kinase protein comprising the homogeneously derived extracellular region, transmembrane regions, and histidine kinase region.

As such a receiver region, usable are, for example, a receiver region of histidine kinase proteins derived from microorganism (e.g. a receiver region of histidine kinase protein derived from a microorganism such as yeast, E. coli) and more particularly receiver region of histidine kinase protein coded in *SLN1* gene derived from budding yeast (e.g. the amino acid sequence represented by SEQ ID No: 7), receiver region of histidine kinase protein coded in Chey gene derived from Salmonella, receiver region of histidine kinase protein coded in *RcsC* gene, which is a hybrid sensor of E. coli [Maeda T, et al. Nature: 369 242-245, (1994): e.g. the amino acid sequence represented by SEQ ID No: 8], receiver region of histidine kinase protein coded in *Phks* gene relevant to cell cycle control of fission yeast [Shieh, JC, et al., Gene Dev. 11, 1008-1022(1997)].

The DNA coding the chimera-type cytokinin receptor may be constructed by respectively producing DNA for each of the extracellular region of the cytokinin receptor, transmembrane regions of the cytokinin receptor, histidine kinase region of the cytokinin receptor, and receiver region for the histidine kinase protein, joining the DNA by a common genetic engineering technique so as to prevent appearance of any termination codon in the middle while using a proper linker so as to prevent frame shift. Incidentally, one DNA fragment may be utilized to provide the DNA encoding the extracellular region of the cytokinin receptor and transmembrane regions of the cytokinin receptor or the DNA encoding the extracellular region of the cytokinin receptor, transmembrane regions of the cytokinin receptor, and histidine kinase region of the cytokinin receptor.

The polynucleotides coding the aforementioned respective regions may respectively be produced by known methods. For example, in the case of production by PCR, at first, the oligonucleotides (5' side primers) having the nucleotide sequences of 5' terminal regions of respective regions to be amplified and oligonucleotides (3' side primers) having complementary nucleotide sequences to the nucleotide sequences of 3' terminal are designed and synthesized. The primers may be oligonucleotides of about 14 nucleotides to about 35 nucleotides in general and are preferable to contain in the 5' side of the primers, restriction enzyme recognition sequences usable at the time of ligating the polynucleotides amplified by the PCR to one another or these polynucleotides to vectors. Then, using the primers and the cDNA library as a template, amplification reactions may be carried out in the common reaction conditions employed for the PCR. As the template to be used in the case of producing the polynucleotides coding the extracellular region and the transmembrane regions of the cytokinin receptor or the histidine kinase region of the cytokinin receptor, usable is cDNA library derived from plants such as higher plants. Also as the template to be used in the case of producing the polynucleotides coding the receiver regions of the histidine kinase, usable is the total DNA or cDNA library derived from microorganism prepared by a common method.

The production of the transformed cell in which the DNA coding the chimera-type cytokinin receptor is introduced may be carried out according to the aforementioned, "Production of the transformed cell in which the DNA coding a cytokinin receptor is introduced.".

### (Intracellular signal transduction system relevant to cytokinin)

Measurement of the existence or the quantity of intracellular signal transduction from the cytokinin receptor expressed in the transformed cell produced by the above-described manner may be carried out by utilizing the intracellular signal transduction system originally present in the host cell used for production of the transformed cell. The existence or the quantity of intracellular signal transduction can be determined by employing the quantity of the cell growth of the transformed cell as an indicator. Alternatively, a regulator (and/or a mediator), which is a downstream signal transducer in the so-called Two-Component regulatory system, can be additionally introduced and expressed in the host cell and the expressed system may be used in the intracellular signal transduction system.
Usable as the Two-Component regulatory system, for example, are Two-Component regulatory systems corresponding to 5 types of ethylene receptors; ETR1, ETR2, ERS1, ERS2, and EIN4; present in Arabidopsis [Chang et al., Science 262: 539-544 (1993), Hua et al., Science 269: 1712-1714 (1995), Sakai et al., Plant Cell Physiol 39: 1232-1239 (1998)] and AtHK1 having sensor functions to the osmotic pressure [Urao, Plant ell 11: 1743-1754 (1999)].

As the host cell to be used for production of such a transformed cell, usable are host cells improved as to have histidine kinase activity lower than the intrinsic histidine kinase activity of the host cells. For example, it includes the host cells improved as to have histidine kinase activity lower than the intrinsic histidine kinase activity of the host cell by deleting one or more of histidine kinases. The phrase, "histidine kinase activity lower than" means that the quantity of a phosphoryl group transfer from Histidine residue (the active site of histidine kinase regions having histidine kinase activity) to Aspartic acid residue (the active site of receiver regions having a reception part) is decreased. Such improvements in the host cell may provide a change of the quantity of the cell growth, change of the morphology, change of the shape, change of the quantity of the biosynthesis of specific compound, change of the quantity of the metabolism of specific compound in the transformed cell. More particularly, the following strain [Maeda T et al., Nature 369: 242-245 (1994)] may be exemplified: a strain obtained by defecting the *SLN1* gene coding the protein having the osmotic pressure sensor function and derived from the budding yeast such as Saccharomyces cerevisiae and the like. Since the strain is defected in the histidine kinase protein existing in Saccharomyces cerevisiae, there can be more clearly detected the existence or the quantity of the intracellular signal transduction from the cytokinin receptor expressed in the transformed cell by using the quantity of the cell growth of the transformed cell as an indicator. Further, other preferable example includes a defective strain of the *Rcs*C gene, which is a hybrid sensor derived from E. coli and a defective strain of *Phks* gene relevant to the cell cycle control of fission yeast.

### (Method for analyzing agonist-activity and antagonist-activity to a cytokinin receptor)

In the method for analyzing agonist-activity to a cytokinin receptor, examples of the first step of bringing an examinee substance into contact with a transformed cell into which DNA coding the cytokinin receptor is introduced include a method for culturing the transformed cell in a culture medium containing the examinee substance. In order to culture the transformed cell, the following cultures are usable: liquid-phase culture for culturing the transformed cell in a liquid culture medium and a solid-phase culture for culturing the transformed cell in a solid culture medium produced by adding agar or the like to the liquid culture medium. The concentration of an examinee substance in the culture medium is about 1 nM to about 1 mM and preferably about 10 nM to about 100 µM. The culture time is, for example, 1 hour to 3 days and preferably 25 hours to 2 days. Incidentally, in the case of method for analyzing the agonist-activity to the cytokinin receptor, the culture containing no cytokinin may be used as the culture containing the examinee substance. When transforming the host cell to analyze the agonist-activity to a cytokinin receptor, it is preferable that the host cell is introduced with DNA encoding any one of the following cytokinin receptors: the cytokinin receptor having the amino acid sequence represented by SEQ ID No: 2; the cytokinin receptor having the amino acid sequence represented by SEQ ID No: 4; the cytokinin receptor wherein said cytokinin receptor has at least one transmembrane region but less than that in its natural form; the cytokinin receptor having the amino acid sequence from amino acid number 196 to 1176 among the amino acid sequence represented by SEQ ID No: 2; the cytokinin receptor having the amino acid sequence from amino acid number 50 to 1176 among the amino acid sequence represented by SEQ ID No: 2; the cytokinin receptor having the amino acid sequence from amino acid number 32 to 1036 among the amino acid sequence represented by SEQ ID No: 4; the chimera-type cytokinin receptor comprising an extracellular region of the cytokinin receptor, transmembrane regions of the cytokinin receptor, a histidine kinase region of the cytokinin receptor, and a receiver region of the histidine kinase, wherein said extracellular region, said transmembrane regions and said histidine kinase region are homogeneous to one another and the receiver region is heterogeneous thereto; and the cytokinin receptor having the amino acid sequence with deletion, substitution, or addition of one or a plurality of amino acids in the amino acid sequence of the above cyctokinin receptors.

In the method for analyzing the antagonist-activity to the cytokinin receptor, examples of the first step of bringing an examinee substance and a substance having the agonist-activity to the cytokinin receptor into contact with a transformed cell into which DNA coding the cytokinin receptor is introduced include a method for culturing the transformed cell in a culture medium containing the examinee substance and a substance having the agonist-activity to the cytokinin receptor. In order to culture the transformed cell, the following cultures are usable: liquid-phase culture for culturing the transformed cell in a liquid culture medium and a solid-phase culture for culturing the transformed cell in a solid culture medium produced by adding agar or the like to the liquid culture medium. The concentration of an examinee substance in the culture medium is about 1 nM to about 1 mM and preferably about 10 nM to about 100 µM. The concentration of the substance (e.g. cytokinins such as trans-zeatin, cis-zeatin, benzyl adenine, or thiadiazuron) : having the agonist-activity to the cytokinin receptor is about 1 nM to about 1 mM and preferably about 10 nM to about 100 µM. The culture time is, for example, 1 hour to 3 days and preferably 25 hours to 2 days. Incidentally, in the case of method for analyzing the antagonist-activity to the cytokinin receptor, the culture containing a cytokinin may be used as the culture medium containing the examinee substance and the substance having the agonist-activity to the cytokinin receptor. When transforming the host cell to analyze the antagonist-activity to a cytokinin receptor, it is preferable that the host cell is introduced with DNA encoding any one of the following cytokinin recpetors: the cytokinin receptor having the amino acid sequence represented by SEQ ID No: 2; the cytokinin receptor having the amino acid sequence represented by SEQ ID No: 4; the cytokinin receptor having the amino acid sequence represented by SEQ ID No: 6; the cytokinin receptor wherein said cytokinin receptor has at least one transmembrane region but less than that in its natural form; the cytokinin receptor having the amino acid sequence from amino acid number 196 to 1176 among the amino acid sequence represented by SEQ ID No: 2; the cytokinin receptor having the amino acid sequence from amino acid number 50 to 1176 among the amino acid sequence represented by SEQ ID No: 2; the cytokinin receptor having the amino acid sequence from amino acid number 32 to 1036 among the amino acid sequence represented by SEQ ID No: 4; the chimera-type cytokinin receptor comprising an extracellular region of the cytokinin receptor, transmembrane regions of the cytokinin receptor, a histidine kinase region of the cytokinin receptor, and a receiver region of the histidine kinase, wherein said extracellular region, said transmembrane regions and said histidine kinase region are homogeneous to one another and the receiver region is heterogeneous thereto; and the cytokinin receptor having the amino acid sequence with deletion, substitution, or addition of one or a plurality of amino acids in the amino acid sequence of the above cyctokinin receptors.

The agonist activity or the antagonist activity of the examinee to the cytokinin receptor can be detected by evaluating the agonist-activity or antagonist-activity of two or more examinee substances. The agonist-activity or the antagonist activity can be provided for the evaluation by conducting the aforementioned method for analyzing the agonist-activity or the antagonist-activity to the cytokinin receptor. For example, the evaluation of the agonist-activity or antagonist activity can be based on the difference between the two or more examinee substances, which are obtained by comparison of the existence or the quantity of the intracellular signal transduction. In utilizing the two or more different examinee substances, the examinee substances are typically utilized independently in different systems. It is preferable that at least one of the two or more examinee substances has no agonist-activity or antagonist-activity to the cytokinin receptor.

More particularly, for example, in the case of using a transformed cell (that is, a transformed cell having a function of directly controlling the cell growth by intracellular signal transduction from the cytokinin receptor) produced using TM182 (*sln1* Δ) [Maeda T et al, Nature 369: 242-245 (1994)], a *SLN1* genetically defected strain in which, for example, *PTP2* Tyrosine phosphatase gene [Ota et al. Proc. N. A. Sci., USA, 89, 2355-2359 (1992)] is introduced as a host cell, the agonist-activity to the cytokinin receptor may be measured using, as an indicator, the quantity of the cell growth of the transformed cell in a culture medium (an agar culture medium or a liquid culture medium) containing glucose as a carbon source, for example, a DOLU + Glu culture medium. In the case of the DOLU + Glu culture medium to which the examinee substance is added, the examinee substance found capable of growing the transformed cell can be evaluated as a substance having the agonist-activity to the cytokinin receptor. On the other hand, in the case of the DOLU+Glu culture medium to which the examinee substance and a substance having the agonist-activity to the cytokinin receptor are added, the examinee substance found capable of suppressing or inhibiting the growth of the transformed cell can be evaluated as a substance having the antagonist-activity to the cytokinin receptor.

Incidentally, as a blank, the growth of the transformed cell in a culture medium using galactose as the carbon source in place of glucose, for example, a DOLU + Gal culture medium, can be investigated independently of the existence of the examinee substance.

Further, in the case of using a transformed cell (that is, a transformed cell having a function of directly controlling the cell growth by the intracellular signal transduction from the cytokinin receptor) produced by employing fission yeast as a host cell, such as a Phks genetically defected strain, the fission pattern of the fission yeast may be observed with a microscope. In this case, when the examinee substance is added to the culture medium containing no substance having the agonist-activity to the cytokinin receptor, the examinee substance found capable of normal fission and propagation of the transformed cell can be evaluated as a substance having the agonist-activity to the cytokinin receptor. On the other hand, if the culture medium contains an examinee substance and a substance having the agonist-activity to the cytokinin receptor, the examinee substance found capable of suppressing or inhibiting the normal fission and propagation of the transformed cell can be evaluated as a substance having the antagonist-activity to the cytokinin receptor.

Furthermore, in the case of using a transformed cell (that is, a transformed cell having a function of directly controlling reporter gene expression by the intracellular signal transduction from the cytokinin receptor) produced by employing as the host cell an E. coli defective in RcsC gene into which cps-LacZ is introduced, the X-Gal coloring may be observed in an agar culture medium or a liquid culture medium [Suzuki et al. Plant Cell Physiol 42: 107-113 (2001)]. In this case, if the culture medium contains the examinee substance and no substance having the agonist-activity to the cytokinin receptor, the examinee substance found capable of coloring the transformed cell to be blue can be evaluated as a substance having the agonist-activity to the cytokinin receptor. On the other hand, if the culture medium contains the examinee substance and a substance having the agonist-activity to the cytokinin receptor, the examinee substance found capable of disappearing coloring the blue of the transformed cell can be evaluated as a substance having the antagonist-activity to the cytokinin receptor.

Moreover, an agonist-active substance or an antagonist-active substance to the cytokinin receptor may be searched by selecting substances having the agonist-active substance or the antagonist-active substance to the cytokinin receptor based on the agonist-activity or the antagonist-activity to the cytokinin receptor evaluated by the aforementioned detection methods.

Further, a substance selected by the above described detection methods may be utilized as an active ingredient of a plant growth regulator.

The plants to be the objects to be treated with the aforementioned plant growth regulator are, for example, decorative plants such as flowering plants and ornamental foliage plants; cultivating plants such as crop; vegetable; fruit; fibrous plants; trees; or lawn.

The growth regulator is generally mixed with, e.g., a solid carrier, a liquid carrier, and further, if needed, mixed with a surfactant and other auxiliary agents for the formulation of an agricultural and horticultural agent and formulated in, e.g., an emulsion agent, a hydrating agent, a suspension agent, or a solution agent. In these agricultural and horticultural agents, an agonist-active substance or an antagonist-active substance to the cytokinin receptor may be contained generally in 0.5 to 90 % by weight and preferably in 1 to 80 % by weight.

Usable as the solid carrier to be used for the formulation of an agricultural and horticultural agent are, for example, clays (e.g., kaolinite, kieselguhr, synthesized hydrated silicon oxide, intercalated clays, bentonite, ^{or} acidic white clay), talc, other inorganic minerals (e.g., sericite, quartz powder, sulfur powder, activated carbon, or calcium carbonate), chemical fertilizers (e.g., ammonium sulphate, ammonium phosphate, ammonium nitrate, ammonium chloride, or urea) in finely powdered state or in granular state and usable as the liquid carrier are, for example, water, alcohols (e.g., methanol, or ethanol), ketones (e.g., acetone, methyl ethyl ketone, or cyclohexanone), aromatic hydrocarbons (e.g., toluene, xylene, ethylbenzene, or methylnaphthalene), non-aromatic hydrocarbons (e.g., hexane, cyclohexane, or kerosene), esters (e.g., ethyl acetate, or butyl acetate), nitriles (e.g., actonitrile, or isobutylnitrile), ethers (e.g., dioxane, or diisopropyl ether), acid amides (e.g., dimethylformamide, or dimethylacetamide), halohydrocarbons (e.g., dichloroethane, or trichloroethane), etc.

As the surfactant, usable are, for example, alkylsulfuric acid esters, alkylsulfonic acid salts, alkylarylsulfonic acid salts, alkylaryl ethers and their polyoxyethylene compounds, polyethylene glycols, polyhydric alcohol esters, or sugaralcohols.

As other auxiliary agents for the formulation of agricultural and horticultural agents, usable are solidification agents and dispersanst such as casein, gelatin, polysaccharides (e.g., starch, acacia, cellulose derivatives, or alginic acid), lignin derivatives, bentonite, or synthesized water-soluble polymer [e.g., poly(vinyl aclcohol), poly(vinyl pyrolidone), or poly(acrylic acid) ] , and stabilizers such as PAP (acidic isopropyl phosphate), BHT (2,6-tert-butyl-4-methylphenol), BHA (2-/3-tert-butyl-4-methoxyphenol), plant oils, mineral oils, aliphatic acids, or aliphatic acid esters.

The agonist-active substance or the antagonist-active substance to the cytokinin receptor made to be agricultural and horticultural agents is used as it is or diluted with water to carry out treatment for the stem and leave parts, branch and leave parts, and flower and fruit parts of plants by spraying, for fruits by immersion, and for fruits by application. The plant growth regulator is used for the object plants to carry out the treatment once or a plurality of times.

In the case of using the plant growth regulator for the purpose of suppressing the dropping of fruits, the plant growth regulator is diluted with water and the resulting diluted agent is sprayed to the fruit parts and branch and leave parts before harvest.

In the case of using the plant growth regulator for the purpose of suppressing the ball dropping of cotton, the plant growth regulator is diluted with water and the resulting diluted agent is sprayed to the balls and stem and leave parts of cotton before harvest.

The plant growth regulator may be used for the treatment of growing plants or of plants after harvest.

The appilcation amount of the agonist-active substance or the antagonist-active substance to the cytokinin receptor in an agricultural and horticultural agent is generally 1 to 8000 g per 1 hectare, although it is changed depending on the state of the agricultural and horticultural agent, the timing for the treatment, the method for the treatment, the site for the treatment, and the object plant to be treated. Also in the case of using the plant growth regulator while diluting the agent with water, the concentration of the agent is generally 0.0001 to 1000 mM and preferably 0.001 to 10 mM, although it is changed depending on the state of the agricultural and horticultural agent, the timing for the treatment, the method for the treatment, the site for the treatment, and the object plant to be treated.

Next, hereinafter given are formulation examples of an agricultural and horticultural agent produced from an agonist-active substance or an antagonist-active substance to the cytokinin receptor and used as a plant growth regulator. The parts in the following description of the examples denotes the parts by weight.

### Formulation example 1

A hydrating agent was obtained by sufficiently pulverizing and mixing 50 parts of an agonist-active substance or an antagonist-active substance to the cytokinin receptor, 3 parts of calcium ligninsulfonate, 2 parts of sodium laurylsulfate, and 45 parts of synthesized hydrated silicon oxide.

### Formulation example 2

A hydrating agent was obtained by sufficiently pulverizing and mixing 70 parts of an agonist-active substance or an antagonist-active substance to the cytokinin receptor, 3 parts of calcium ligninsulfonate, 2 parts of sodium laurylsulfate, and 25 parts of synthesized hydrated silicon oxide.

### Formulation example 3

An emulsion agent was obtained by mixing 40 parts of an agonist-active substance or an antagonist-active substance to the cytokinin receptor, 3 parts of polyoxyethylene sorbitane monoolate, 2 parts of CMC (carboxymethyl cellulose), and 52 parts of water and wet-pulverizing the resultant mixture to be 5 µm or smaller in the particle size.

The present invention makes it possible to analyze the agonist-activity and the antagonist-activity to the cytokinin receptor and also makes it possible to quickly search the substances having the agonist-activity and the antagonist-activity to the cytokinin receptor even in a small amount of examinee substances by employing the analysis method.

### EXAMPLES

Hereinafter, although the present invention will be described in details with the reference to examples, the present invention is not at all restricted to these examples.

### (Example 1) Production of Arabidopsis cDNA phage library for CRE1 cloning

Seeds of Arabidopsis thaliana ecotype Wassilewskija were sterilized with 70% of ethyl alcohol for 1 minute and further sterilized with 1.5% of sodium hypochlorite for 10 minutes. The resulting seeds were well washed with sterilized water and then cultured for 2 weeks in GM culture medium [4.3 g Murashige and Skoog's basal salt mixture, 1% sucrose, 10 ml of 5% MES-KOH (pH 5.7), 0.3% Phytagel^{™} (SIGMA)] to obtain 5g of the plant. After the plant was frozen in liquefied nitrogen and physically milled with a mortar and a pestle. The resulting milled product was mixed with a mixed solution of 10 ml of an extraction buffer [200 mM Tris-HCl (pH 8.5), 100 mM NaCl, 10 mM EDTA, 0.5% SDS, 14 mM β-mercaptoethanol] and 10 g of phenol. After being mixed by a Vortex mixer, the resulting mixture was mixed further with 10 ml of chloroform and vigorously stirred and subjected to centrifugal separation at 10,000 rotation for 20 minutes. The recovered aqueous layer was mixed with LiCl in the concentration to be 2M of the final concentration, left still at -80°C for 3 hours, thawed and subjected to centrifugal separation at 10,000 rotation for 20 minutes to recover a precipitate. The recovered precipitate was dissolved in 2 ml of TE [10 mM Tris-HCl (pH 8.0), 1 mM EDTA] and then further mixed with 0.2 ml of 3 M sodium acetate (pH 5.2) and 5 ml ethanol and subjected to centrifugal separation to recover RNA as a precipitate. Further, the precipitate (RNA) was subjected to treatment with Oligotex^{™} dT30super (Nippon Rosch Co.) to extract RNA integrated with polyA.

The production of phage cDNA library from the extracted RNA integrated with polyA was carried out employing ZAP-cDNAR Synthesis Kit (Stratagene Co.) according to the instruction. The potency of the produced phage cDNA library was 500,000 PFU.

### (Example 2) Production of DNA probe of CRE1

The PCR was carried out employing TAKARA LA Taq^{™} (Takara Shuzo Co., Ltd.) and using a phage solution (about 1,000,000 PFU) of the phage cDNA library produced in the example 1 as a template and DNA having the nucleotide sequence represented by SEQ ID No: 11 and DNA having the nucleotide sequence represented by SEQ ID No: 12 as the primers to amplify DNA. The procedure will be described in details below.

A PCR solution was prepared by adding a reaction composition containing dNTP and the like to the phage 1,000,000 pft and respective primer DNA each in 0.2 µM according to the instruction of the kit and the desired DNA fragment was amplified in PCR conditions: keeping at 94°C for 2 minutes, and further repeating 40 cycles each of which comprised keeping at 94°C for 30 seconds, at 55°C for 30 seconds, and at 68°C for 5 minute. Next, using the amplified DNA fragment as a template, a probe labelled with ³²P was prepared employing Megaprime DNA-labelling system kit (Amersham Pharmacia Co.). Incidentally, reaction solution (25 µl) was prepared by adding 2.0 MBq of ³²P dCTP to 25 ng of amplified DNA fragment and adding a reaction composition instructed by the kit. The labelling reaction was carried out at 37°C for 10 minutes.

### (Example 3) Production of phage cDNA clone holding CRE 1

The cloning of desired CRE 1 gene was carried out by plaque hybridization using the probe prepared by the example 2. Detailed description will be given below.

Using the phage cDNA library produced in the example 1 and according to the instruction of ZAP-cDNAR Synthesis Kit, plaque was produced. DNA was adsorbed on a nitrocellulose filter from the produced plaque and then treated with UV rays to be fixed on the filter. Using the filter prepared in such a manner, hybridized phage cDNA clone was obtained by keeping at 65°C in the presence of 6 × SSC (0.9 M NaCl and 0.09 M trisodium citrate), 5 × Denhard's solution [0.1% (w/v) ficoll 400, 0.1% (w/v) polyvinylpyrrolidone, 0.1% BSA], 0.5% (w/v) SDS and 100 µg/ml degenerated salmon sperm DNA or in DIG EASY Hyb solution (Boeringer-Mannheim Co.) containing 100 µg/ml of degenerated salmon sperm DNA, successively keeping at a room temperature for 15 minutes two times in the presence of 1 × SSC (0.15 M NaCl and 0.015 M trisodium citrate) and 0.5% (w/v) SDS, and further keeping at 68°C for 30 minutes in the presence of 0.1 × SSC (0.015 M NaCl and 0.0015 M trisodium citrate) and 0.5% SDS.

### (Example 4) Cloning of CRE 1 cDNA

Using the cDNA of the phage cDNA clone obtained by the example 3 as a template and DNA having the nucleotide sequence represented by SEQ ID No: 13 and DNA represented by SEQ ID No: 14 as primers, DNA having the nucleotide sequence represented by SEQ ID No: 5 was amplified by PCR. Detailed description will be given below.

The PCR was carried out employing Herculase Enhanced DNA Polymerase (TOYOBO Co., Ltd.) in the reaction conditions of keeping at 94°C for 1 minute, and further repeating 25 cycles each of which comprised keeping at 94°C for 30 seconds, at 55°C for 30 seconds, and at 72°C for 4 minute. Incidentally, the PCR solution (50 µ 1) was prepared by adding a reaction composition containing dNTP and the like to 500 ng of cDNA of the phage cDNA clone and respective primer DNA each in 100 ng according to the instruction of the kit.

The desired DNA fragment was amplified in such a manner.

### (Example 5) Construction of CRE 1 expression vector

After p415CYC1, a yeast expression vector, [Munberg et al. Gene: 156 119-122 (1995), available from ATCC library (No. 873821)] was digested with a restriction enzyme Sma I and then using T4 DNA ligase, DNA having the nucleotide sequence represented by SEQ ID No: 5 and obtained by the example 4 was ligated downstream of the CYC 1 promoter sequence of the expression vector p415CYC1 as to be integrated to express the desired protein in yeast. The constructed DNA was confirmed to be in the right direction and its nucleotide sequence was confirmed to be the nucleotide sequence represented by SEQ ID No: 5 by an automatic DNA sequencer and then the expression plasmid p415CYC-CRE 1 was obtained.

### (Example 6) Cloning of AHK3 (AAF99730) cDNA

Seeds of Arabidopsis thaliana ecotype Wassilewskija were sterilized with 70% of ethyl alcohol for 1 minute and further sterilized with 1.5% of sodium hypochlorite for 10 minutes. The resulting seeds were well washed with sterilized water and then cultured for 2 weeks in GM culture medium [4.3 g Murashige and Skoog's basal salt mixture, 1% sucrose, 10 ml of 5% MES-KOH (pH 5.7), 0.3% Phytagel^{™} (SIGMA)] to obtain 5g of the plant. After the plant was frozen in liquefied nitrogen and physically milled with a mortar and a pestle. The resulting milled product was mixed with a mixed solution of 10 ml of an extraction buffer [200 mM Tris-HCl (pH 8.5), 100 mM NaCl, 10 mM EDTA, 0.5% SDS, 14 mM β-mercaptoethanol] and 10 g of phenol. After being mixed by a Voltex mixer, the resulting mixture was mixed further with 10 ml of chloroform and vigorously stirred and subjected to centrifugal separation at 10,000 rotation for 20 minutes. The recovered aqueous layer was mixed with LiCl in the concentration to be 2M of the final concentration, left still at -80°C for 3 hours, thawed and subjected to centrifugal separation at 10,000 rotation for 20 minutes to recover a precipitate. The recovered precipitate was dissolved in 2 ml of TE [10 mM Tris-HCl (pH 8.0), 1 mM EDTA] and then further mixed with 0.2 ml of 3 M sodium acetate (pH 5.2) and 5 ml ethanol and subjected to centrifugal separation to recover RNA as a precipitate. Further, 40 µg of the precipitate (RNA) was mixed with 30 unit of FPLC pure^{™} DnaseI (Amersham-Pharmacia) and 60 unit of Superace (Ambion) to remove mixed genome DNA and the resulting RNA was subjected to the phenol/chloroform treatment and ethanol treatment to purify the RNA. Next, using the purified RNA as a template and oligo (dT) 12-18 (Amersham-Pharmacia) as a primer, RT-PCR was carried out. The RT-PCR was carried out employing Superscript II (GIBCO BRL Co.) at 42°C for 40 minutes. Incidentally the PT-PCR solution was prepared according to the method described in instruction of the Superscript II.

The desired cDNA was amplified in such a manner.

Using the amplified cDNA as a template and DNA having the nucleotide sequence represented by SEQ ID No: 16 and DNA having the nucleotide sequence represented by SEQ ID No: 17 as primers, DNA having the nucleotide sequence represented by SEQ ID No: 3 was amplified by PCR. The PCR was carried out using Herculase Enhanced DNA Polymerase (TOYOBO Co., Ltd.) in the reaction conditions of keeping at 94°C for 1 minute, and further repeating 41 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of the template DNA and respective primer DNA each in 100 ng according to the instruction of the kit.

The desired DNA fragment was amplified in such a manner.

### (Example 7) Construction of pHM-1

After p415CYC1 [Munberg et al. Gene: 156 119-122 (1995); available from ATCC library (No. 87382)] was digested with the restriction enzyme Spe I and BamH I, synthesized DNA fragments (linkers) having the nucleotide sequence represented by SEQ ID Nos: 18 and 15 were inserted to the expression vector p415CYC1 using T4 DNA ligaseas to newly add the restriction enzyme sites Sac II, Apa I, Nhe I to the plasmid p415CYC1 and construct pHM-1.

### (Example 8) Construction of AHK3 expression vector

After the pHM-1 was digested with the restriction enzyme Sal I and Sac II, and then using T4 DNA ligase, DNA having the nucleotide sequence represented by SEQ ID No: 3 was introduced downstream of the CYC 1 promoter sequence of the expression vector pHM-1 as to be integrated to express the desired protein in yeast. The constructed DNA was confirmed to be right in the direction and its nucleotide sequence was confirmed to be right in the sequence by an automatic DNA sequencer and thus the expression plasmid p415CYC-AHK3 was obtained.

### (Example 9) Cloning of AHK2(BAB09274)cDNA

Using the cDNA prepared in the example 6 (the cDNA prepared by reverse transcription of the total RNA) as a template and DNA having the nucleotide sequence represented by SEQ ID No: 19 and DNA having the nucleotide sequence represented by SEQ ID No: 20 as primers, DNA having the nucleotide sequence represented by SEQ ID No: 1 was amplified by PCR. The PCR was carried out employing TAKARA Pfu Turbo denature (Takara Shuzo Co., Ltd.) in the reaction conditions of keeping at 94°C for 1 minute, and further repeating 30 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of the template DNA and respective primer DNA each in 50 ng according to the instruction of the kit.

The desired DNA fragment was amplified in such a manner.

### (Example 10) Cloning of AHK2(BAB09274) Δ cDNA

Using the cDNA prepared in example 6 (the cDNA prepared by reverse transcription of the total RNA) as a template and DNA having the nucleotide sequence represented by SEQ ID No: 21 and DNA having the nucleotide sequence represented by SEQ ID No: 22 as primers, PCR was carried out to amplify DNA having the nucleotide sequence represented by SEQ ID No: 1 in which ATG was added to the 5' terminal sites of the nucleotide sequence from the nucleotide numbers 586 to 3531. The PCR was carried out employing TAKARA Pfu Turbo denature (Takara Shuzo Co., Ltd.) in the reaction conditions of keeping at 94°C for 1 minute, and further repeating 30 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of the template DNA and respective primer DNA each in 50 ng according to the instruction of the kit.

The desired DNA fragment was amplified in such a manner.

### (Example 11) Construction of AHK2 and AHK2 Δ expression vectors

After pHM-1 was digested with a restriction enzyme Sac II and Nhe I and then using T4 DNA ligase, DNA fragments obtained by the example 9 and the example 10 were respectively ligated downstream to the CYC 1 promoter sequence of the expression vector pHM-1 as to be integrated to express the desired protein in yeast. The introduced DNA was confirmed to be right in the direction and its nucleotide sequence was confirmed to be right in the sequence by an automatic DNA sequencer and thus the expression plasmid p415CYC-AHK 2 and p415CYC-AHK2 Δ were obtained.

### (Example 12) Production of transformed cells TM182-CRE1, TM182-AHK2, TM182-AHK2 Δ and TM182-AHK3

Transformation of TM182 (*sln1* Δ)*, SLN1* genetically detected strain, [Maeda T et al. Nature: 369 242-245 (1994)] was carried out using the obtained expression plasmid p415CYC-CRE1 (the example 5), p415CYC-AHK2 (the example 11), p415CYC-AHK2 Δ (the example 11) and p415CYC-AHK3 (the example 8). The transformation was carried out by employing Polyethylene glycol/lithium acetate (PEG/LiAc)-mediated transformation method according to the description of VII. Library Transformation & Screening Protocols disclosed in MATCHAMAKER Two-Hybrid System 3 User Manual p. 22 from CLONTECH Co. Transformed cells TM182-CRE1, TM182-AHK2, TM182-AHK2 Δ and TM182-AHK3 were produced by selecting in the DOLU + Gal culture medium the transformed cells having a disappearance of leucine nutrient requirement.

### (Example 13) Response of transformed cells TM182-CRE1, TM182-AHK2, TM182-AHK2Δ and TM182-AHK3 to cytokinin (Part 1)

A culture solution 10 µl (about 800 clones of yeast) of the transformed cells TM182-CRE1,TM 182-AHK2, TM182-AHK2 Δ and TM182-AHK3 produced by the example 12 was spotted on DOLU + Glu agar media containing 10 µM of trans-zeatin and cultured at 30°Cfor 30 hours. After incubation, the growth state of the transformed cells was observed and photographed by a digital camera.

As a result, each of the transformed cells TM182-CRE1, TM182-AHK2, TM182-AHK2 Δ and TM182-AHK3 showed high growth in the case of using the DOLU + Glu agar culture media containing trans-zeatin as compared with that in the case of using the DOLU + Glu agar culture media containing no trans-zeatin. The results showed that the transformed cells responded to cytokinin and that they were possible to be grown in the DOLU + Glu agar culture media. Further the transformed cells were found possible to be grown in the DOLU + Gal agar culture media independently of the existence trans-zeatin.

### (Example 14) Response of transformed cells TM182-CRE1, TM182-AHK2Δ and TM182-AHK2 to cytokinin (Part 2)

Culture solutions of the transformed cells TM182-CRE1, TM182-AHK2 Δ and TM182-AHK2 produced by the example 12 were spotted on DOLU + Glu agar media containing cytokinin in a variety of concentrations (1 nM, 10 nM, 100 nM, 1 µM, 10 µM, 100 µM) and cultured at 30°C for 30 hours. After incubation, the growth state of the transformed cells was observed and photographed by a digital camera.

The lowest observed supply concentrations of trans-zeatin and cis-zeatin in which the respective transformed cells could be grown were shown in Table 1.

**Table 1**

| | | | |
|---|---|---|---|
| cytokinin | TM182-CRE1 | TM182-AHK2 | TM182-AHK2 Δ |
| trans-zeatin | 0µM | 1µM | 100nM |
| cis-zeatin | no growth | 10µM | 1µM |

### (Example 15) Method for searching substance having agonist-activity to cytokinin receptor (Part 1)

The transformed cells TM182-CRE1,TM 182-AHK2, TM182-AHK2 Δ and TM182-AHK3 produced in the example 12 were inoculated in 200 ml of DOLU + Gal culture media and pre-cultured at 30°C for 36 hours. The pre-cultured solutions were diluted with DOLU + Glu media as to become 0.100 as the optical density (OD₆₀₀) and further the resultants were diluted with DOLU + Glu media at a dilution rate of 1/200 to obtain diluted pre-cultured solutions.

An assay plate was prepared by filling respective wells of a 96-well plate with 20 µl of solutions which were prepared by diluting DMSO solution (10mM) of each examinee substance with DOLU + Glu medium at a dilution rate of 1/100, said solutions containing each examinee substance to be 100 µM at the final concentration. Simultaneously, an assay plate only filled with 20 µl of solutions which were prepared by diluting DMSO solution with DOLU + Glu medium, said solutions containing no examinee substance, was prepared as the blank sections.

The diluted pre-cultured solutions were added in 200 µl each into the respective wells of both assay plates and cultured at 30°C for 24 hours and then the optical density (OD₆₂₀) of each well was measured by a plate reader. The agonist-activity of the examinee substances to the cytokinin receptor was detected by comparing the optical density measured in the testing sections to which the examinee substances were added with the optical density measured in the blank sections. The optical density of the culture solutions of the transformed cells in the testing sections to which the examinee substances were added were shown in Table 2. Compound B showing the higher optical density measured in the testing sections to which the examinee substances were added than that in the blank sections were selected as agonist-active substance to the cytokinin receptor.

**Table 2**

| Examinee substance | TM182-AHK 2 | TM182-AHK 2 Δ | TM182-AHK 3 |
|---|---|---|---|
| DMSO | 0.01 | 0 | 0.51 |
| Compound A^{*1} | 0.01 | 0 | 0.54 |
| Compound B*² | 0.45 | 0.89 | 0.88 |

| | | | |
|---|---|---|---|
| ^{*1} Abscisic acid (=negative control) ^{*2} 6-Benzyl aminopurine (=positive control) | | | |

### (Example 16) Method for searching substance having antagonist-activity to cytokinin receptor (Part 2)

The transformed cells TM182-CRE1, TM182-AHK2, TM 182-AHK2 Δ and TM182-AHK3 produced in the example 12 are inoculated in 200 ml of DOLU + Gal culture media and cultured at 30°C for 30 hours to obtain pre-cultured solutions.

An assay plate is prepared by filling respective wells of a 96-well plate with 200 µl of each DOLU + Glu medium mixed with 1 µM of trans-zeatin (cytokinin), and into the assay plate is put each examinee substance to be 1 µM at the final concentration. Simultaneously, an assay plate only filled with 200 µl of the DOLU + Gal medium mixed with 1 µM of trans-zeatin is prepared as the blank sections

The pre-cultured solutions are added in 20 µl each into the respective wells of both assay plates and cultured at 30°C for 30 hours and then the optical density of each well is measured by a plate reader. The antagonist-activity of the examinee substances to the cytokinin receptor is detected by comparing the optical density measured in the testing section to which the examinee substances are added with the optical density measured in the blank sections. The examinee substances showing the lower optical density measured in the testing sections to which the examinee substances are added than that in the blank sections are selected as antagonist-active substance to the cytokinin receptor.

### (Example 17) Method for searching substance having agonist-activity to cytokinin receptor (Part 3)

The transformed cells TM182-CRE1, TM182-AHK2, TM182-AHK2Δ and TM182-AHK3 produced in the example 12 are inoculated in 200 ml of DOLU + Gal culture media and cultured at 30°C for 30 hours to obtain pre-cultured solutions.

The pre-cultured transformed cells (TM182-CRE1, TM182-AHK2, TM182-AHK2 Δ and TM182-AHK3) are spot-added in 10 µl to the respective DOLU + Glu agar culture media to which agonist-active substances to the cytokinin receptor selected by the example 15 are added while their concentration being changed from 10 nM to 100 µM and then are cultured at 30°C for 30 hours. After incubation, the intensity of the agonist-activity of the examinee substances to the cytokinin receptor is detected and confirmed based on the lowest concentration at which transformed cells (TM182-CRE1,TM182-AHK2, TM182-AHK2 Δ and TM182-AHK3) are observed to grow.

### (Example 18) Method for searching substance having antagonist-activity to cytokinin receptor (Part 4)

The transformed cells TM182-CRE1,TM182-AHK2, TM182-AHK2 Δ and TM182-AHK3 produced in the example 12 are inoculated in 200 ml of DOLU + Gal culture media and cultured at 30°C for 30 hours to obtain pre-cultured solutions.

The pre-cultured transformed cells (TM182-CRE1, TM182-AHK2, TM182-AHK2 Δ and TM182-AHK3) are spot-added in 10 µl to the respective DOLU + Gul agar culture media to which antagonist-active substances to the cytokinin receptor selected by the example 16 are added while their concentration being changed from 10 nM to 100 µM and also 10 µM of trans-zeatin (cytokinin) is added and then is cultured at 30°C for 30 hours. After incubation, the intensity of the antagonist-activity of the examinee substances to the cytokinin receptor is detected and confirmed based on the lowest concentration at which transformed cells (TM182-CRE1, TM182-AHK2, TM182-AHK2 Δ and TM182-AHK3) are not observed to grow.

Hereinafter, the medium compositions to be used in the present invention will be described:
(a) DOLU + Glu culture medium

| | |
|---|---|
| Bacto-yeast nitrogen base without amino acids | 6.7g |
| Glucose | 20g |
| Drop-out mix (x) | 2.0g |
| Distilled water | 1000ml |

(b)DOLU + GAL culture medium

| | |
|---|---|
| Bacto-yeast nitrogen base without amino acids | 6.7g |
| Glucose | 20g |
| Drop-out mix (x) | 2.0g |
| Distilled water | 1000m1 |

(x) Drop-out mix: Drop-out mix is a combination of the following ingredients.

| | | | |
|---|---|---|---|
| Adenine | 0.5g | Lysine | 2.0g |
| Alanine | 2.0g | Methionine | 2.0g |
| Arginine | 2.0g | para-Aminobenzoic acid | 0.2g |
| Asparagine | 2.0g | Phenylalanine | 2.0g |
| Aspartic acid | 2.0g | Proline | 2.0g |
| Cysteine | 2.0g | Serine | 2.0g |
| Glutamine | 2.0g | Threonine | 2.0g |
| Glutamic acid | 2.0g | Trytophan | 2.0g |
| Glycine | 2.0g | Tyrosine | 2.0g |
| Histidine | 2.0g | Valine | 2.0g |
| Inositol | 2.0g | Isoleucine | 2.0g |

(c) DOLU + Glu agar culture medium
A solid culture medium prepared by adding 2% (W/V) of agar into the culture medium (a)
(d) DOLU + GAL agar culture medium
A solid culture medium prepared by adding 2% (W/V) of agar into the culture medium (c)

### " Sequence table free text"

SEQ ID No: 9
   Oligonucleotide primer designed for PCR
SEQ ID No: 10
   Oligonucleotide primer designed for PCR
SEQ ID No: 11
   Oligonucleotide primer designed for PCR
SEQ ID No: 12
   Oligonucleotide primer designed for PCR
SEQ ID No: 13
   Oligonucleotide primer designed for PCR
SEQ ID No: 14
   Oligonucleotide primer designed for PCR
SEQ ID No: 15
   Oligonucleotide primer designed for PCR
SEQ ID No: 16
   Oligonucleotide primer designed for PCR
SEQ ID No: 17
   Oligonucleotide primer designed for PCR
SEQ ID No: 18
   Oligonucleotide primer designed for PCR
SEQ ID No: 19
   Oligonucleotide primer designed for PCR
SEQ ID No: 20
   Oligonucleotide primer designed for PCR
SEQ ID No: 21
   Oligonucleotide primer designed for PCR
SEQ ID No: 22
   Oligonucleotide primer designed for PCR

## Claims

1. A method for analyzing antagonist-activity to a cytokinin receptor, which comprises
(1) culturing a transformed cell into which a DNA encoding a cytokinin receptor is introduced in a liquid culture medium containing an examinee substance and a substance having agonist-activity to the cytokinin receptor, and
(2) measuring an existence or quantity of intracellular signal transduction from the cytokinin receptor expressed in the transformed cell,
wherein growth of the transformed cell is controlled by intracellular signal transduction from the cytokinin receptor, and wherein the existence or the quantity of the intracellular signal transduction is determined by measuring growth of the transformed cell, and
wherein the substance having agonist-activity is selected from the group consisting of: trans-zeatin, cis-zeatin, benzyl adenine and thidiazuron.

2. A method for analyzing agonist-activity to a cytokinin receptor, which comprises
(1) culturing a transformed cell into which a DNA encoding a cytokinin receptor is introduced in a liquid culture medium containing an examinee substance; and
(2) measuring the existence or the quantity of intracellular signal transduction from the cytokinin receptor expressed in the transformed cell,
wherein growth of the transformed cell is controlled by intracellular signal transduction from the cytokinin receptor, and wherein the existence or the quantity of the intracellular signal transduction is determined by measuring growth of the transformed cell.

3. The method of claim 1 or 2, wherein the transformed cell is a transformed cell generated by introducing DNA encoding the cytokinin receptor into a host cell so improved as to have histidine kinase activity lower than the intrinsic histidine kinase activity of the host cell.

4. The method of any one of claims 1 to 3, wherein the transformed cell is a transformed cell generated by introducing DNA encoding the cytokinin receptor into a host cell so improved as to have histidine kinase activity lower than the intrinsic histidine kinase activity of the host cell by deleting one or more of histidine kinase.

5. The method of claim 1 or 2, wherein the transformed cell is a transformed cell generated by introducing DNA encoding the cytokinin receptor into a host cell having no cytokinin receptor.

6. The method of any one of claims 1 to 5, wherein the transformed cell is yeast.

7. The method of claim 6, wherein the transformed cell is budding yeast.

8. A method for detecting antagonist-activity to a cytokinin receptor, which comprises evaluating the antagonist-activity of two or more different examinee substances to the cytokinin receptor based on the difference obtained by comparison of the existence or the quantity of the intracellular signal transduction in a section where the examinee substances are independently used and measured by the analysis method of claim 1.

9. The method of claim 8, wherein at least one substance among the two or more different examinee substances is a substance having no antagonist-activity to the cytokinin receptor.

10. A method for detecting agonist-activity to a cytokinin receptor, which comprises evaluating the agonist-activity of two or more different examinee substances to the cytokinin receptor based on the difference obtained by comparison of the existence or the quantity of the intracellular signal transduction in a section where the examinee substances are independently used and measured by the analysis method of claim 2.

11. The method of claim 10, wherein at least one substance among the two or more different examinee substances is a substance having no agonist-activity to the cytokinin receptor.

## Patentansprüche

1. Verfahren zum Analysieren von Antagonistenaktivität gegenüber einem Cytokininrezeptor, das umfasst:
(1) Züchten einer transformierten Zelle, in die eine DNA, die einen Cytokininrezeptor codiert, eingeführt wird, in einem flüssigen Kulturmedium, das eine zu prüfende Substanz und eine Substanz mit Agonistenaktivität gegenüber dem Cytokininrezeptor enthält; und
(2) Messen des Vorhandenseins oder der Menge der intrazellulären Signaltransduktion, die vom Cytokininrezeptor, der in der transformierten Zelle exprimiert wird, ausgeht,
wobei das Wachstum der transformierten Zelle durch die intrazelluläre Signaltransduktion, die vom Cytokininrezeptor ausgeht, kontrolliert wird, und wobei das Vorhandensein oder die Menge der intrazellulären Signaltransduktion durch das Messen des Wachstums der transformierten Zelle bestimmt wird, und
wobei die Substanz, die Agonistenaktivität aufweist, ausgewählt ist aus der Gruppe bestehend aus: trans-Zeatin, cis-Zeatin, Benzyladenin und Thidiazuron.

2. Verfahren zum Analysieren von Agonistenaktivität gegenüber einem Cytokininrezeptor, das umfasst:
(1) Züchten einer transfomierten Zelle, in die eine DNA, die einen Cytokininrezeptor codiert, eingeführt wird, in einem flüssigen Kulturmedium, das eine zu prüfende Substanz enthält; und
(2) Messen des Vorhandenseins oder der Menge der intrazellulären Signaltransduktion, die vom Cytokininrezeptor, der in der transformierten Zelle exprimiert wird, ausgeht,
wobei das Wachstum der transformierten Zelle durch die intrazelluläre Signaltransduktion, die vom Cytokininrezeptor ausgeht, kontrolliert wird, und wobei das Vorhandensein oder die Menge der intrazellulären Signaltransduktion durch das Messen des Wachstums der transformierten Zelle bestimmt wird.

3. Verfahren nach Anspruch 1 oder 2, wobei die transformierte Zelle eine transformierte Zelle ist, die durch Einführen von DNA, die den Cytokininrezeptor codiert, in eine Wirtszelle erzeugt wird, die so verbessert wurde, dass sie eine Histidinkinaseaktivität aufweist, die geringer ist als die intrinsische Histidinkinaseaktivität der Wirtszelle.

4. Verfahren nach einem der Ansprüche 1 bis 3, wobei die transformierte Zelle eine transformierte Zelle ist, die durch Einführen von DNA, die den Cytokininrezeptor codiert, in eine Wirtszelle erzeugt wird, die so verbessert wurde, dass sie durch Deletion einer oder mehrerer Histidinkinase(n) eine Histidinkinaseaktivität aufweist, die geringer ist als die intrinsische Histidinkinaseaktivität der Wirtszelle.

5. Verfahren nach Anspruch 1 oder 2, wobei die transformierte Zelle eine transformierte Zelle ist, die durch Einführen von DNA, die den Cytokininrezeptor codiert, in eine Wirtszelle, die keinen Cytokininrezeptor aufweist, hergestellt wird.

6. Verfahren nach einem der Ansprüche 1 bis 5, wobei die transformierte Zelle Hefe ist.

7. Verfahren nach Anspruch 6, wobei die transformierte Zelle Sprosshefe ist.

8. Verfahren zum Nachweisen von Antagonistenaktivität gegenüber einem Cytokininrezeptor, das die Evaluierung der Antagonistenaktivität von zwei oder mehreren verschiedenen zu prüfenden Substanzen gegenüber dem Cytokininrezeptor umfasst, auf der Basis des Unterschieds, der durch Vergleich des Vorhandenseins oder der Menge der intrazellulären Signaltransduktion in einem Abschnitt ermittelt wird, in dem die zu prüfenden Substanzen unabhängig voneinander verwendet und gemäß dem Analyseverfahren nach Anspruch 1 gemessen werden.

9. Verfahren nach Anspruch 8, wobei mindestens eine Substanz der zwei oder mehreren verschiedenen zu prüfenden Substanzen eine Substanz ist, die gegenüber dem Cytokininrezeptor keine Antagonistenaktivität aufweist.

10. Verfahren zum Nachweisen von Agonistenaktivität gegenüber einem Cytokininrezeptor, das die Evaluierung der Agonistenaktivität von zwei oder mehreren verschiedenen zu prüfenden Substanzen gegenüber dem Cytokininrezeptor umfasst, auf der Basis des Unterschieds, der durch Vergleich des Vorhandenseins oder der Menge der intrazellulären Signaltransduktion in einem Abschnitt ermittelt wird, in dem die zu prüfenden Substanzen unabhängig voneinander verwendet und gemäß dem Analyseverfahren nach Anspruch 2 gemessen werden.

11. Verfahren nach Anspruch 10, wobei mindestens eine der zwei oder mehreren verschiedenen zu prüfenden Substanzen eine Substanz ist, die gegenüber dem Cytokininrezeptor keine Agonistenaktivität aufweist.

## Revendications

1. Procédé pour analyser une activité d'antagoniste à un récepteur de cytokinine, qui comprend les étapes consistant à :
(1) réaliser une culture d'une cellule transformée dans laquelle un ADN encodant un récepteur de cytokinine est introduit dans un milieu de culture liquide contenant une substance examinée et une substance possédant une activité d'agoniste au récepteur de cytokinine ; et
(2) mesurer une existence ou une quantité de transduction de signal intracellulaire à partir du récepteur de cytokinine exprimé dans la cellule transformée,
dans lequel la croissance de la cellule transformée est contrôlée par transduction de signal intracellulaire à partir du récepteur de cytokinine, et dans lequel l'existence ou la quantité de la transduction de signal intracellulaire est déterminée en mesurant la croissance de la cellule transformée, et
dans lequel la substance possédant une activité d'agoniste est sélectionnée parmi le groupe constitué de : trans-zéatine, cis-zéatine, benzyladénine et thidiazurone.

2. Procédé pour analyser une activité d'agoniste à un récepteur de cytokinine, qui comprend les étapes consistant à :
(1) réaliser une culture d'une cellule transformée dans laquelle un ADN encodant un récepteur de cytokinine est introduit dans un milieu de culture liquide contenant une substance examinée ; et
(2) mesurer l'existence ou la quantité de transduction de signal intracellulaire à partir du récepteur de cytokinine exprimé dans la cellule transformée,
dans lequel la croissance de la cellule transformée est contrôlée par transduction de signal intracellulaire à partir du récepteur de cytokinine, et dans lequel l'existence ou la quantité de la transduction de signal intracellulaire est déterminée en mesurant la croissance de la cellule transformée.

3. Procédé selon la revendication 1 ou 2, dans lequel la cellule transformée est une cellule transformée générée en introduisant un ADN encodant le récepteur de cytokinine dans une cellule hôte améliorée afin de posséder une activité d'histidine kinase inférieure à l'activité d'histidine kinase intrinsèque de la cellule hôte.

4. Procédé selon une quelconque des revendications 1 à 3, dans lequel la cellule transformée est une cellule transformée générée en introduisant un ADN encodant le récepteur de cytokinine dans une cellule hôte améliorée afin de posséder une activité d'histidine kinase inférieure à l'activité d'histidine kinase intrinsèque de la cellule hôte en supprimant une ou plusieurs des histidine kinases.

5. Procédé selon la revendication 1 ou 2, dans lequel la cellule transformée est une cellule transformée générée en introduisant un ADN encodant le récepteur de cytokinine dans une cellule hôte ne possédant aucun récepteur de cytokinine.

6. Procédé selon une quelconque des revendications 1 à 5, dans lequel la cellule transformée est de la levure.

7. Procédé selon la revendication 6, dans lequel la cellule transformée est de la levure de bourgeonnement.

8. Procédé pour détecter une activité d'antagoniste à un récepteur de cytokinine, qui comprend l'étape consistant à évaluer l'activité d'antagoniste de deux substances examinées différentes, ou plus, au récepteur de cytokinine en fonction de la différence obtenue par comparaison de l'existence ou de la quantité de la transduction de signal intracellulaire dans une section où les substances examinées sont indépendamment utilisées et mesurées par le procédé d'analyse selon la revendication 1.

9. Procédé selon la revendication 8, dans lequel au moins une substance parmi les deux substances examinées différentes, ou plus, est une substance ne possédant aucune activité d'antagoniste au récepteur de cytokinine.

10. Procédé pour détecter une activité d'agoniste à un récepteur de cytokinine, qui comprend l'étape consistant à évaluer l'activité d'agoniste de deux substances examinées différentes, ou plus, au récepteur de cytokinine en fonction de la différence obtenue par comparaison de l'existence ou de la quantité de la transduction de signal intracellulaire dans une section où les substances examinées sont indépendamment utilisées et mesurées par le procédé d'analyse selon la revendication 2.

11. Procédé selon la revendication 10, dans lequel au moins une substance parmi les deux substances examinées différentes, ou plus, est une substance ne possédant aucune activité d'agoniste au récepteur de cytokinine.


### SEQUENCE LISTING

<110> Sumitomo Chemical Co., Ltd.
<120> Analysis of agonist-activity and antagonist-activity to cytokinin receptor
<130> G 1374 EP
<160> 22
<170> PatentIn Ver. 2.1
<210> 1
   <211> 3531
   <212> DNA
   <213> Arabidopsis thaliana
<220>
   <221> CDS
   <222> (1)..(3531)
<400> 1
   atg tct ata act tgt gag ctc ttg aat ctt act tca aag aaa gct aag     48
   Met Ser Ile Thr Cys Glu Leu Leu Asn Leu Thr Ser Lys Lys Ala Lys
     1               5                  10                  15
   aag tcg tcg agc agt gac aag aaa tgg cta aag aag cct ctc ttc ttc     96
   Lys Ser Ser Ser Ser Asp Lys Lys Trp Leu Lys Lys Pro Leu Phe Phe
                20                  25                  30
   ctg att ttg tgt ggc tct ttg gta att gtt ttg gtt atg ttc tta cgg    144
   Leu Ile Leu Cys Gly Ser Leu Val Ile Val Leu Val Met Phe Leu Arg
            35                  40                  45
   tta ggt aga agt cag aag gag gag aca gat tct tgt aat gga gaa gag    192
   Leu Gly Arg Ser Gln Lys Glu Glu Thr Asp Ser Cys Asn Gly Glu Glu
        50                  55                  60
   aaa gtg ttg tat aga cat caa aat gtc aca aga agt gag att cat gat    240
   Lys Val Leu Tyr Arg His Gln Asn Val Thr Arg Ser Glu Ile His Asp
    65                  70                  75                  80
   ttg gtc tct ttg ttc tct gat tca gat cag gta aca tcc ttt gaa tgt    288
   Leu Val Ser Leu Phe Ser Asp Ser Asp Gln Val Thr Ser Phe Glu Cys
                    85                  90                  95
   cat aag gaa tca agc cct gga atg tgg aca aac tat ggt att aca tgt    336
   His Lys Glu Ser Ser Pro Gly Met Trp Thr Asn Tyr Gly Ile Thr Cys
               100                 105                 110
   tcc ctg agt gtg cgt tct gat aaa caa gag act aga ggg ctt ccc tgg    384
   Ser Leu Ser Val Arg Ser Asp Lys Gln Glu Thr Arg Gly Leu Pro Trp
           115                 120                 125
   aat ctt ggc tta gga cat tct atc tca tca aca tct tgt atg tgt ggt    432
   Asn Leu Gly Leu Gly His Ser Ile Ser Ser Thr Ser Cys Met Cys Gly
       130                 135                 140
   aat ctt gaa ccg att tta cag caa cct gaa aac ctt gag gaa gaa aac    480
   Asn Leu Glu Pro Ile Leu Gln Gln Pro Glu Asn Leu Glu Glu Glu Asn
   145                 150                 155                 160
   cat gaa gaa ggg ctg gag cag ggt ttg tca tcg tat tta aga aat gca    528
   His Glu Glu Gly Leu Glu Gln Gly Leu Ser Ser Tyr Leu Arg Asn Ala
                   165                 170                 175
   tgg tgg tgt cta atc ctt ggt gtg tta gtg tgc cat aag att tat gta      576
   Trp Trp Cys Leu Ile Leu Gly Val Leu Val Cys His Lys Ile Tyr Val
               180                 185                 190
   tct cat tct aaa gca cga ggt gag agg aaa gag aaa gta cat ctg caa      624
   Ser His Ser Lys Ala Arg Gly Glu Arg Lys Glu Lys Val His Leu Gln
           195                 200                 205
   gag gct tta gct cca aag aag cag caa caa cgt gct cag act tct tct      672
   Glu Ala Leu Ala Pro Lys Lys Gln Gln Gln Arg Ala Gln Thr Ser Ser
       210                 215                 220
   aga ggg gct gga aga tgg agg aag aat atc ctt ctc ctt ggt att tta      720
   Arg Gly Ala Gly Arg Trp Arg Lys Asn Ile Leu Leu Leu Gly Ile Leu
   225                 230                 235                 240
   gga gga gtt tcc ttc tct gtt tgg tgg ttt tgg gac act aat gag gag      768
   Gly Gly Val Ser Phe Ser Val Trp Trp Phe Trp Asp Thr Asn Glu Glu
                   245                 250                 255
   atc ata atg aaa agg agg gag act ttg gca aac atg tgt gac gaa cga      816
   Ile Ile Met Lys Arg Arg Glu Thr Leu Ala Asn Met Cys Asp Glu Arg
               260                 265                 270
   gca cgt gtt tta caa gat cag ttc aat gtt agc ttg aac cat gtt cat      864
   Ala Arg Val Leu Gln Asp Gln Phe Asn Val Ser Leu Asn His Val His
           275                 280                 285
   gcc ttg tct att ctt gta tct aca ttt cat cat ggt aaa atc cca tct      912
   Ala Leu Ser Ile Leu Val Ser Thr Phe His His Gly Lys Ile Pro Ser
       290                 295                 300
   gcc att gat cag aga aca ttt gaa gaa tat act gag aga aca aac ttt      960
   Ala Ile Asp Gln Arg Thr Phe Glu Glu Tyr Thr Glu Arg Thr Asn Phe
   305                 310                 315                 320
   gag agg cca ctt act agt ggt gta gcg tat gct ttg aaa gtc cca cac     1008
   Glu Arg Pro Leu Thr Ser Gly Val Ala Tyr Ala Leu Lys Val Pro His
                   325                 330                 335
   tca gaa aga gag aaa ttt gaa aag gag cat gga tgg gca ata aag aaa     1056
   Ser Glu Arg Glu Lys Phe Glu Lys Glu His Gly Trp Ala Ile Lys Lys
               340                 345                 350
   atg gaa act gag gac cag aca gtt gta caa gat tgt gtt cct gaa aac     1104
   Met Glu Thr Glu Asp Gln Thr Val Val Gln Asp Cys Val Pro Glu Asn
           355                 360                 365
   ttt gat ccc gca ccg att caa gac gaa tac gcg cca gtt ata ttt gct     1152
   Phe Asp Pro Ala Pro Ile Gln Asp Glu Tyr Ala Pro Val Ile Phe Ala
       370                 375                 380
   caa gaa act gtt tcc cat att gta tcg gtc gac atg atg tct gga gaa     1200
   Gln Glu Thr Val Ser His Ile Val Ser Val Asp Met Met Ser Gly Glu
   385                 390                 395                 400
   gaa gac cgt gaa aac atc tta cgg gca agg gca tca gga aaa gga gtg     1248
   Glu Asp Arg Glu Asn Ile Leu Arg Ala Arg Ala Ser Gly Lys Gly Val
                   405                 410                 415
   tta aca tct cca ttt aag ctt ctt aag tca aat cat ctt ggt gtt gtg    1296
   Leu Thr Ser Pro Phe Lys Leu Leu Lys Ser Asn His Leu Gly Val Val
               420                 425                 430
   ttg acc ttt gct gtc tat gac acg agc cta ccg cct gat gct aca gaa    1344
   Leu Thr Phe Ala Val Tyr Asp Thr Ser Leu Pro Pro Asp Ala Thr Glu
           435                 440                 445
   gaa cag cgt gtt gaa gca act att ggg tac ctt ggt gca tca tat gat    1392
   Glu Gln Arg Val Glu Ala Thr Ile Gly Tyr Leu Gly Ala Ser Tyr Asp
       450                 455                 460
   atg cca tcg ctg gtg gag aaa ctt ctt cac caa ctt gcc agc aaa cag    1440
   Met Pro Ser Leu Val Glu Lys Leu Leu His Gln Leu Ala Ser Lys Gln
   465                 470                 475                 480
   aca att gct gtg gat gtt tac gac aca act aac act tca ggt cta ata    1488
   Thr Ile Ala Val Asp Val Tyr Asp Thr Thr Asn Thr Ser Gly Leu Ile
                   485                 490                 495
   aaa atg tat ggc tca gaa att ggg gat ata agt gag cag cat ata agt    1536
   Lys Met Tyr Gly Ser Glu Ile Gly Asp Ile Ser Glu Gln His Ile Ser
               500                 505                 510
   agc ctt gat ttt ggt gat cca tca agg aac cat gag atg cat tgc agg    1584
   Ser Leu Asp Phe Gly Asp Pro Ser Arg Asn His Glu Met His Cys Arg
           515                 520                 525
   ttt aag cat aaa ctt ccc att ccc tgg aca gcg ata aca ccg tcg atc    1632
   Phe Lys His Lys Leu Pro Ile Pro Trp Thr Ala Ile Thr Pro Ser Ile
       530                 535                 540
   tta gtt ctg gtt att act ttt ctt gtt ggt tat att tta tat gaa gcc    1680
   Leu Val Leu Val Ile Thr Phe Leu Val Gly Tyr Ile Leu Tyr Glu Ala
   545                 550                 555                 560
   atc aac cga att gcg aca gtt gaa gag gat tgt cag aag atg agg gaa    1728
   Ile Asn Arg Ile Ala Thr Val Glu Glu Asp Cys Gln Lys Met Arg Glu
                   565                 570                 575
   ctc aaa gct cgt gct gag gcc gct gac att gca aag tca cag ttc cta    1776
   Leu Lys Ala Arg Ala Glu Ala Ala Asp Ile Ala Lys Ser Gln Phe Leu
               580                 585                 590
   gca act gtt tct cat gag ata cgg act ccg atg aat gga gtt tta gga    1824
   Ala Thr Val Ser His Glu Ile Arg Thr Pro Met Asn Gly Val Leu Gly
           595                 600                 605
   atg ctg aaa atg ctg atg gac acc gat ctt gat gcg aag cag atg gac    1872
   Met Leu Lys Met Leu Met Asp Thr Asp Leu Asp Ala Lys Gln Met Asp
       610                 615                 620
   tat gcg caa act gct cat ggc agt ggg aag gat ctt aca tca cta ata    1920
   Tyr Ala Gln Thr Ala His Gly Ser Gly Lys Asp Leu Thr Ser Leu Ile
   625                 630                 635                 640
   aat gag gtt ctt gat cag gca aag att gaa tcc gga agg ctc gag ctt    1968
   Asn Glu Val Leu Asp Gln Ala Lys Ile Glu Ser Gly Arg Leu Glu Leu
                   645                 650                 655
   gaa aat gtg cct ttt gat atg cgt ttt att ctt gat aat gtt tca tct    2016
   Glu Asn Val Pro Phe Asp Met Arg Phe Ile Leu Asp Asn Val Ser Ser
               660                 665                 670
   ctc ctc tct ggc aag gca aat gaa aaa gga att gag ttg gcc gtt tat   2064
   Leu Leu Ser Gly Lys Ala Asn Glu Lys Gly Ile Glu Leu Ala Val Tyr
           675                 680                 685
   gtt tct agt caa gtt cct gat gtt gta gtc ggt gat ccg agt cgg ttc   2112
   Val Ser Ser Gln Val Pro Asp Val Val Val Gly Asp Pro Ser Arg Phe
       690                 695                 700
   cgg cag atc att aca aac ctg gtt gga aac tca atc aaa ttc aca cag   2160
   Arg Gln Ile Ile Thr Asn Leu Val Gly Asn Ser Ile Lys Phe Thr Gln
   705                 710                 715                 720
   gaa agg gga cac ata ttt atc tca gtg cac ctt gca gat gag gta aag   2208
   Glu Arg Gly His Ile Phe Ile Ser Val His Leu Ala Asp Glu Val Lys
                   725                 730                 735
   gag cct ctt act att gaa gac gca gtg cta aaa cag cga cta gct tta   2256
   Glu Pro Leu Thr Ile Glu Asp Ala Val Leu Lys Gln Arg Leu Ala Leu
               740                 745                 750
   gga tgc agc gag tcc ggt gag aca gtt agc ggg ttt cct gcg gta aat   2304
   Gly Cys Ser Glu Ser Gly Glu Thr Val Ser Gly Phe Pro Ala Val Asn
           755                 760                 765
   gca tgg gga agc tgg aag aat ttc aag aca tgt tac agt act gag agt   2352
   Ala Trp Gly Ser Trp Lys Asn Phe Lys Thr Cys Tyr Ser Thr Glu Ser
       770                 775                 780
   cag aat tct gat caa atc aaa ttg cta gtt aca gtg gag gac act gga   2400
   Gln Asn Ser Asp Gln Ile Lys Leu Leu Val Thr Val Glu Asp Thr Gly
   785                 790                 795                 800
   gtt ggc ata cct gtg gat gca caa ggc cga atc ttc aca cct ttt atg   2448
   Val Gly Ile Pro Val Asp Ala Gln Gly Arg Ile Phe Thr Pro Phe Met
                   805                 810                 815
   caa gcc gac agt tcc aca tcg cgg act tat ggt gga act ggc ata ggt   2496
   Gln Ala Asp Ser Ser Thr Ser Arg Thr Tyr Gly Gly Thr Gly Ile Gly
               820                 825                 830
   ttg agt ata agc aaa cgt ttg gtt gaa ctc atg caa gga gag atg ggg   2544
   Leu Ser Ile Ser Lys Arg Leu Val Glu Leu Met Gln Gly Glu Met Gly
           835                 840                 845
   ttt gtg agt gag ccc ggg ata ggc agt act ttt tca ttt act gga gtt   2592
   Phe Val Ser Glu Pro Gly Ile Gly Ser Thr Phe Ser Phe Thr Gly Val
       850                 855                 860
   ttc ggg aaa gca gaa aca aat acg tcg att act aag ctg gaa cga ttc   2640
   Phe Gly Lys Ala Glu Thr Asn Thr Ser Ile Thr Lys Leu Glu Arg Phe
   865                 870                 875                 880
   gat cta gct att cag gag ttt aca gga ttg aga gca tta gtt att gat   2688
   Asp Leu Ala Ile Gln Glu Phe Thr Gly Leu Arg Ala Leu Val Ile Asp
                   885                 890                 895
   aac aga aac att aga gca gag gtc acc agg tac gaa ctt cgg aga ctg   2736
   Asn Arg Asn Ile Arg Ala Glu Val Thr Arg Tyr Glu Leu Arg Arg Leu
               900                 905                 910
   gga ata tct gca gac att gtt tca agt ctg aga atg gca tgc act tgt   2784
   Gly Ile Ser Ala Asp Ile Val Ser Ser Leu Arg Met Ala Cys Thr Cys
               915                     920                     925
   tgt atc agc aaa tta gaa aat ttg gct atg att cta ata gac aaa gac      2832
   Cys Ile Ser Lys Leu Glu Asn Leu Ala Met Ile Leu Ile Asp Lys Asp
           930                     935                     940
   gcc tgg aac aag gaa gaa ttt tca gta ctt gac gag ttg ttt acc cga      2880
   Ala Trp Asn Lys Glu Glu Phe Ser Val Leu Asp Glu Leu Phe Thr Arg
   945                     950                     955                     960
   agc aaa gta acc ttt aca aga gtc cca aag att ttt ctt ttg gca act      2928
   Ser Lys Val Thr Phe Thr Arg Val Pro Lys Ile Phe Leu Leu Ala Thr
                   965                     970                     975
   tct gca act ctt act gag cgc agt gag atg aag tct act ggt ctc atc      2976
   Ser Ala Thr Leu Thr Glu Arg Ser Glu Met Lys Ser Thr Gly Leu Ile
               980                     985                     990
   gat gag gtg gtg ata aag cct ctt cgg atg agt gtc tta ata tgt tgc      3024
   Asp Glu Val Val Ile Lys Pro Leu Arg Met Ser Val Leu Ile Cys Cys
           995                    1000                    1005
   ttg caa gaa acc ctt gtc aat ggc aag aag agg caa ccg aac aga cag      3072
   Leu Gln Glu Thr Leu Val Asn Gly Lys Lys Arg Gln Pro Asn Arg Gln
       1010                    1015                    1020
   cga aga aat ctt gga cac ttg cta aga gaa aaa cag att ctg gtt gtg      3120
   Arg Arg Asn Leu Gly His Leu Leu Arg Glu Lys Gln Ile Leu Val Val
   1025                    1030                    1035                    1040
   gat gat aat ctt gtg aac aga cga gtt gca gaa ggt gca ctt aag aaa      3168
   Asp Asp Asn Leu Val Asn Arg Arg Val Ala Glu Gly Ala Leu Lys Lys
                   1045                    1050                    1055
   tat gga gct att gtt aca tgc gtt gag agt ggc aaa gct gca ttg gca      3216
   Tyr Gly Ala Ile Val Thr Cys Val Glu Ser Gly Lys Ala Ala Leu Ala
               1060                    1065                    1070
   atg ctt aag ccg cct cat aac ttc gat gct tgc ttc atg gat ctc cag      3264
   Met Leu Lys Pro Pro His Asn Phe Asp Ala Cys Phe Met Asp Leu Gln
           1075                    1080                    1085
   atg cct gaa atg gat gga ttt gaa gcg aca agg aga gtc cgt gag ctg      3312
   Met Pro Glu Met Asp Gly Phe Glu Ala Thr Arg Arg Val Arg Glu Leu
       1090                    1095                    1100
   gag agg gaa atc aat aag aaa ata gct tct gga gaa gtt tca gct gaa      3360
   Glu Arg Glu Ile Asn Lys Lys Ile Ala Ser Gly Glu Val Ser Ala Glu
   1105                    1110                    1115                    1120
   atg ttc tgt aaa ttt agt agt tgg cac gtc ccg ata tta gca atg aca      3408
   Met Phe Cys Lys Phe Ser Ser Trp His Val Pro Ile Leu Ala Met Thr
                   1125                    1130                    1135
   gca gat gtt att cag gct act cat gaa gaa tgc atg aaa tgt gga atg      3456
   Ala Asp Val Ile Gln Ala Thr His Glu Glu Cys Met Lys Cys Gly Met
               1140                    1145                    1150
   gat ggt tat gta tca aaa ccg ttt gaa gag gaa gtg ctc tac aca gcg      3504
   Asp Gly Tyr Val Ser Lys Pro Phe Glu Glu Glu Val Leu Tyr Thr Ala
           1155                    1160                    1165
   gta gca aga ttc ttt gaa cct tgt taa                                  3531
   Val Ala Arg Phe Phe Glu Pro Cys
   1170 1175
<210> 2
   <211> 1176
   <212> PRT
   <213> Arabidopsis thaliana
<400> 2
   Met Ser Ile Thr Cys Glu Leu Leu Asn Leu Thr Ser Lys Lys Ala Lys
    1               5                  10                  15
   Lys Ser Ser Ser Ser Asp Lys Lys Trp Leu Lys Lys Pro Leu Phe Phe
               20                  25                  30
   Leu Ile Leu Cys Gly Ser Leu Val Ile Val Leu Val Met Phe Leu Arg
           35                  40                  45
   Leu Gly Arg Ser Gln Lys Glu Glu Thr Asp Ser Cys Asn Gly Glu Glu
       50                  55                  60
   Lys Val Leu Tyr Arg His Gln Asn Val Thr Arg Ser Glu Ile His Asp
   65                  70                  75                  80
   Leu Val Ser Leu Phe Ser Asp Ser Asp Gln Val Thr Ser Phe Glu Cys
                   85                  90                  95
   His Lys Glu Ser Ser Pro Gly Met Trp Thr Asn Tyr Gly Ile Thr Cys
               100                 105                 110
   Ser Leu Ser Val Arg Ser Asp Lys Gln Glu Thr Arg Gly Leu Pro Trp
           115                 120                 125
   Asn Leu Gly Leu Gly His Ser Ile Ser Ser Thr Ser Cys Met Cys Gly
       130                 135                 140
   Asn Leu Glu Pro Ile Leu Gln Gln Pro Glu Asn Leu Glu Glu Glu Asn
   145                 150                 155                 160
   His Glu Glu Gly Leu Glu Gln Gly Leu Ser Ser Tyr Leu Arg Asn Ala
                   165                 170                 175
   Trp Trp Cys Leu Ile Leu Gly Val Leu Val Cys His Lys Ile Tyr Val
               180                 185                 190
   Ser His Ser Lys Ala Arg Gly Glu Arg Lys Glu Lys Val His Leu Gln
           195                 200                 205
   Glu Ala Leu Ala Pro Lys Lys Gln Gln Gln Arg Ala Gln Thr Ser Ser
       210                 215                 220
   Arg Gly Ala Gly Arg Trp Arg Lys Asn Ile Leu Leu Leu Gly Ile Leu
   225                 230                 235                 240
   Gly Gly Val Ser Phe Ser Val Trp Trp Phe Trp Asp Thr Asn Glu Glu
                   245                 250                 255
   Ile Ile Met Lys Arg Arg Glu Thr Leu Ala Asn Met Cys Asp Glu Arg
               260                 265                 270
   Ala Arg Val Leu Gln Asp Gln Phe Asn Val Ser Leu Asn His Val His
           275                 280                 285
   Ala Leu Ser Ile Leu Val Ser Thr Phe His His Gly Lys Ile Pro Ser
       290                 295                 300
   Ala Ile Asp Gln Arg Thr Phe Glu Glu Tyr Thr Glu Arg Thr Asn Phe
   305                 310                 315                 320
   Glu Arg Pro Leu Thr Ser Gly Val Ala Tyr Ala Leu Lys Val Pro His
                   325                 330                 335
   Ser Glu Arg Glu Lys Phe Glu Lys Glu His Gly Trp Ala Ile Lys Lys
               340                 345                 350
   Met Glu Thr Glu Asp Gln Thr Val Val Gln Asp Cys Val Pro Glu Asn
           355                 360                 365
   Phe Asp Pro Ala Pro Ile Gln Asp Glu Tyr Ala Pro Val Ile Phe Ala
       370                 375                 380
   Gln Glu Thr Val Ser His Ile Val Ser Val Asp Met Met Ser Gly Glu
   385                 390                 395                 400
   Glu Asp Arg Glu Asn Ile Leu Arg Ala Arg Ala Ser Gly Lys Gly Val
                   405                 410                 415
   Leu Thr Ser Pro Phe Lys Leu Leu Lys Ser Asn His Leu Gly Val Val
                       420                 425                 430
   Leu Thr Phe Ala Val Tyr Asp Thr Ser Leu Pro Pro Asp Ala Thr Glu
               435                 440                 445
   Glu Gln Arg Val Glu Ala Thr Ile Gly Tyr Leu Gly Ala Ser Tyr Asp
           450                 455                 460
   Met Pro Ser Leu Val Glu Lys Leu Leu His Gln Leu Ala Ser Lys Gln
   465                 470                 475                 480
   Thr Ile Ala Val Asp Val Tyr Asp Thr Thr Asn Thr Ser Gly Leu Ile
                   485                 490                 495
   Lys Met Tyr Gly Ser Glu Ile Gly Asp Ile Ser Glu Gln His Ile Ser
               500                 505                 510
   Ser Leu Asp Phe Gly Asp Pro Ser Arg Asn His Glu Met His Cys Arg
           515                 520                 525
   Phe Lys His Lys Leu Pro Ile Pro Trp Thr Ala Ile Thr Pro Ser Ile
       530                 535                 540
   Leu Val Leu Val Ile Thr Phe Leu Val Gly Tyr Ile Leu Tyr Glu Ala
   545                 550                 555                 560
   Ile Asn Arg Ile Ala Thr Val Glu Glu Asp Cys Gln Lys Met Arg Glu
                   565                 570                 575
   Leu Lys Ala Arg Ala Glu Ala Ala Asp Ile Ala Lys Ser Gln Phe Leu
               580                 585                 590
   Ala Thr Val Ser His Glu Ile Arg Thr Pro Met Asn Gly Val Leu Gly
           595                 600                 605
   Met Leu Lys Met Leu Met Asp Thr Asp Leu Asp Ala Lys Gln Met Asp
       610                 615                 620
   Tyr Ala Gln Thr Ala His Gly Ser Gly Lys Asp Leu Thr Ser Leu Ile
   625                 630                 635                 640
   Asn Glu Val Leu Asp Gln Ala Lys Ile Glu Ser Gly Arg Leu Glu Leu
                   645                 650                 655
   Glu Asn Val Pro Phe Asp Met Arg Phe Ile Leu Asp Asn Val Ser Ser
               660                 665                 670
   Leu Leu Ser Gly Lys Ala Asn Glu Lys Gly Ile Glu Leu Ala Val Tyr
           675                 680                 685
   Val Ser Ser Gln Val Pro Asp Val Val Val Gly Asp Pro Ser Arg Phe
       690                 695                 700
   Arg Gln Ile Ile Thr Asn Leu Val Gly Asn Ser Ile Lys Phe Thr Gln
   705                 710                 715                 720
   Glu Arg Gly His Ile Phe Ile Ser Val His Leu Ala Asp Glu Val Lys
                   725                 730                 735
   Glu Pro Leu Thr Ile Glu Asp Ala Val Leu Lys Gln Arg Leu Ala Leu
               740                 745                 750
   Gly Cys Ser Glu Ser Gly Glu Thr Val Ser Gly Phe Pro Ala Val Asn
           755                 760                 765
   Ala Trp Gly Ser Trp Lys Asn Phe Lys Thr Cys Tyr Ser Thr Glu Ser
       770                 775                 780
   Gln Asn Ser Asp Gln Ile Lys Leu Leu Val Thr Val Glu Asp Thr Gly
   785                 790                 795                 800
   Val Gly Ile Pro Val Asp Ala Gln Gly Arg Ile Phe Thr Pro Phe Met
                   805                 810                 815
   Gln Ala Asp Ser Ser Thr Ser Arg Thr Tyr Gly Gly Thr Gly Ile Gly
               820                 825                 830
   Leu Ser Ile Ser Lys Arg Leu Val Glu Leu Met Gln Gly Glu Met Gly
           835                 840                 845
   Phe Val Ser Glu Pro Gly Ile Gly Ser Thr Phe Ser Phe Thr Gly Val
       850                 855                 860
   Phe Gly Lys Ala Glu Thr Asn Thr Ser Ile Thr Lys Leu Glu Arg Phe
   865                 870                 875                 880
   Asp Leu Ala Ile Gln Glu Phe Thr Gly Leu Arg Ala Leu Val Ile Asp
                   885                 890                 895
   Asn Arg Asn Ile Arg Ala Glu Val Thr Arg Tyr Glu Leu Arg Arg Leu
               900                 905                 910
   Gly Ile Ser Ala Asp Ile Val Ser Ser Leu Arg Met Ala Cys Thr Cys
           915                 920                 925
   Cys Ile Ser Lys Leu Glu Asn Leu Ala Met Ile Leu Ile Asp Lys Asp
   930 935 940
   Ala Trp Asn Lys Glu Glu Phe Ser Val Leu Asp Glu Leu Phe Thr Arg
   945 950 955 960
   Ser Lys Val Thr Phe Thr Arg Val Pro Lys Ile Phe Leu Leu Ala Thr
   965 970 975
   Ser Ala Thr Leu Thr Glu Arg Ser Glu Met Lys Ser Thr Gly Leu Ile
   980 985 990
   Asp Glu Val Val Ile Lys Pro Leu Arg Met Ser Val Leu Ile Cys Cys
   995 1000 1005
   Leu Gln Glu Thr Leu Val Asn Gly Lys Lys Arg Gln Pro Asn Arg Gln
   1010 1015 1020
   Arg Arg Asn Leu Gly His Leu Leu Arg Glu Lys Gln Ile Leu Val Val
   1025 1030 1035 1040
   Asp Asp Asn Leu Val Asn Arg Arg Val Ala Glu Gly Ala Leu Lys Lys
   1045 1050 1055
   Tyr Gly Ala Ile Val Thr Cys Val Glu Ser Gly Lys Ala Ala Leu Ala
   1060 1065 1070
   Met Leu Lys Pro Pro His Asn Phe Asp Ala Cys Phe Met Asp Leu Gln
   1075 1080 1085
   Met Pro Glu Met Asp Gly Phe Glu Ala Thr Arg Arg Val Arg Glu Leu
   1090 1095 1100
   Glu Arg Glu Ile Asn Lys Lys Ile Ala Ser Gly Glu Val Ser Ala Glu
   1105 1110 1115 1120
   Met Phe Cys Lys Phe Ser Ser Trp His Val Pro Ile Leu Ala Met Thr
   1125 1130 1135
   Ala Asp Val Ile Gln Ala Thr His Glu Glu Cys Met Lys Cys Gly Met
   1140 1145 1150
   Asp Gly Tyr Val Ser Lys Pro Phe Glu Glu Glu Val Leu Tyr Thr Ala
   1155 1160 1165
   Val Ala Arg Phe Phe Glu Pro Cys
   1170 1175
<210> 3
   <211> 3111
   <212> DNA
   <213> Arabidopsis thaliana
<220>
   <221> CDS
   <222> (1)..(3111)
<400> 3
   atg agt ctg ttc cat gtg cta ggg ttt ggt gtc aag att ggg cat ctc    48
   Met Ser Leu Phe His Val Leu Gly Phe Gly Val Lys Ile Gly His Leu
    1               5                  10                  15
   ttc tgg atg cta tgc tgc tgg ttt gtt tct tgg ttc gtt gat aat ggg    96
   Phe Trp Met Leu Cys Cys Trp Phe Val Ser Trp Phe Val Asp Asn Gly
               20                  25                  30
   atc gag gac aag tct ggt ctt tta gtt ggc tct gtc ggt gat ctt gag   144
   Ile Glu Asp Lys Ser Gly Leu Leu Val Gly Ser Val Gly Asp Leu Glu
           35                  40                  45
   aag act aag atg act acg ttg aag aag aag aac aag atg tgg ttc tgg   192
   Lys Thr Lys Met Thr Thr Leu Lys Lys Lys Asn Lys Met Trp Phe Trp
       50                  55                  60
   aat aag atc tct agc agc gga ctc aag atc ccg agt ttc tct tat cag   240
   Asn Lys Ile Ser Ser Ser Gly Leu Lys Ile Pro Ser Phe Ser Tyr Gln
    65                  70                  75                  80
   ttt ctt ggc tct gtt aaa ttc aac aag gcg tgg tgg agg aag ctt gtg     288
   Phe Leu Gly Ser Val Lys Phe Asn Lys Ala Trp Trp Arg Lys Leu Val
                   85                  90                  95
   gtg gtt tgg gtt gtc ttc tgg gtc ttg gtc tct att tgg acg ttt tgg     336
   Val Val Trp Val Val Phe Trp Val Leu Val Ser Ile Trp Thr Phe Trp
               100                 105                 110
   tac ttt agc tcg caa gct atg gag aag agg aaa gag acg cta gct agt     384
   Tyr Phe Ser Ser Gln Ala Met Glu Lys Arg Lys Glu Thr Leu Ala Ser
           115                 120                 125
   atg tgt gat gag aga gct cgt atg ctg cag gat cag ttc aac gtt agc     432
   Met Cys Asp Glu Arg Ala Arg Met Leu Gln Asp Gln Phe Asn Val Ser
       130                 135                 140
   atg aat cat gtt caa gcc atg tct atc ttg atc tca acc ttc cac cat     480
   Met Asn His Val Gln Ala Met Ser Ile Leu Ile Ser Thr Phe His His
   145                 150                 155                 160
   ggc aag att cct tct gct atc gat cag aga aca ttc tca gag tac act     528
   Gly Lys Ile Pro Ser Ala Ile Asp Gln Arg Thr Phe Ser Glu Tyr Thr
                   165                 170                 175
   gat aga act tcc ttt gag agg cct ctt act agc ggg gta gct tat gct     576
   Asp Arg Thr Ser Phe Glu Arg Pro Leu Thr Ser Gly Val Ala Tyr Ala
               180                 185                 190
   atg agg gtg ctc cat tca gag agg gaa gag ttc gag agg caa caa ggt     624
   Met Arg Val Leu His Ser Glu Arg Glu Glu Phe Glu Arg Gln Gln Gly
           195                 200                 205
   tgg act att agg aag atg tat tct ctt gaa caa aac cca gtt cac aag     672
   Trp Thr Ile Arg Lys Met Tyr Ser Leu Glu Gln Asn Pro Val His Lys
       210                 215                 220
   gat gac tat gac ctg gaa gct ttg gaa cca tcc cct gtc caa gaa gag     720
   Asp Asp Tyr Asp Leu Glu Ala Leu Glu Pro Ser Pro Val Gln Glu Glu
   225                 230                 235                 240
   tac gct cca gtc atc ttt gct cag gac act gtt tct cac gtt gtt tct     768
   Tyr Ala Pro Val Ile Phe Ala Gln Asp Thr Val Ser His Val Val Ser
                   245                 250                 255
   ctc gat atg ctg tct ggg aaa gaa gat cgt gaa aac gtt ttg cgg gcc     816
   Leu Asp Met Leu Ser Gly Lys Glu Asp Arg Glu Asn Val Leu Arg Ala
               260                 265                 270
   agg agt tca ggt aaa ggg gtt ttg aca gct cct ttc cca ttg ata aag     864
   Arg Ser Ser Gly Lys Gly Val Leu Thr Ala Pro Phe Pro Leu Ile Lys
           275                 280                 285
   aca aat aga ctt ggg gtg atc ctg aca ttt gca gtg tac aag aga gat     912
   Thr Asn Arg Leu Gly Val Ile Leu Thr Phe Ala Val Tyr Lys Arg Asp
       290                 295                 300
   ctc ccc tcc aat gca acg cca aaa gag aga att gag gct act aac ggg     960
   Leu Pro Ser Asn Ala Thr Pro Lys Glu Arg Ile Glu Ala Thr Asn Gly
   305                 310                 315                 320
   tat ctc ggg gga gtg ttt gac att gag tcc ctg gta gaa aac ttg ctt    1008
   Tyr Leu Gly Gly Val Phe Asp Ile Glu Ser Leu Val Glu Asn Leu Leu
                   325                 330                 335
   caa cag ctg gct agc aag caa acg att ctt gtc aat gtg tac gat atc    1056
   Gln Gln Leu Ala Ser Lys Gln Thr Ile Leu Val Asn Val Tyr Asp Ile
               340                 345                 350
   acc aat cac tct caa ccg att agc atg tat ggt aca aat gtg tcg gct    1104
   Thr Asn His Ser Gln Pro Ile Ser Met Tyr Gly Thr Asn Val Ser Ala
           355                 360                 365
   gat ggg ttg gaa cgt gtt agt cca cta atc ttt ggc gat cca ttg aga    1152
   Asp Gly Leu Glu Arg Val Ser Pro Leu Ile Phe Gly Asp Pro Leu Arg
       370                 375                 380
   aag cat gag atg cgt tgc aga ttt aag cag aaa cca cca tgg cca gtg    1200
   Lys His Glu Met Arg Cys Arg Phe Lys Gln Lys Pro Pro Trp Pro Val
   385                 390                 395                 400
   cta tca atg gtg aca tca ttc ggt atc ctt gtg att gcg tta ctt gtt    1248
   Leu Ser Met Val Thr Ser Phe Gly Ile Leu Val Ile Ala Leu Leu Val
                   405                 410                 415
   gca cat ata atc cac gca acc gtt agt cga ata cac aaa gtt gaa gaa    1296
   Ala His Ile Ile His Ala Thr Val Ser Arg Ile His Lys Val Glu Glu
               420                 425                 430
   gat tgt gat aaa atg aag cag ctc aag aaa aag gct gaa gca gca gat    1344
   Asp Cys Asp Lys Met Lys Gln Leu Lys Lys Lys Ala Glu Ala Ala Asp
           435                 440                 445
   gtt gca aag tca cag ttc ctt gcc act gtt tca cat gaa atc aga act    1392
   Val Ala Lys Ser Gln Phe Leu Ala Thr Val Ser His Glu Ile Arg Thr
       450                 455                 460
   cca atg aat ggt gtt cta gga atg ttg cat atg ctt atg gac aca gag    1440
   Pro Met Asn Gly Val Leu Gly Met Leu His Met Leu Met Asp Thr Glu
   465                 470                 475                 480
   tta gat gtt acg caa cag gat tat gtt agg acc gca cag gca agt gga    1488
   Leu Asp Val Thr Gln Gln Asp Tyr Val Arg Thr Ala Gln Ala Ser Gly
                   485                 490                 495
   aaa gct tta gtc tcg cta ata aat gag gtt ttg gac caa gca aag att    1536
   Lys Ala Leu Val Ser Leu Ile Asn Glu Val Leu Asp Gln Ala Lys Ile
               500                 505                 510
   gaa tct gga aag ctt gaa ctt gag gag gtg cgg ttt gat ttg aga gga    1584
   Glu Ser Gly Lys Leu Glu Leu Glu Glu Val Arg Phe Asp Leu Arg Gly
           515                 520                 525
   ata tta gat gat gtc ctg tca ctc ttc tct agc aag tcc caa caa aag    1632
   Ile Leu Asp Asp Val Leu Ser Leu Phe Ser Ser Lys Ser Gln Gln Lys
       530                 535                 540
   ggg gtg gag ttg gca gta tac ata tct gat cgt gtt cca gat atg tta    1680
   Gly Val Glu Leu Ala Val Tyr Ile Ser Asp Arg Val Pro Asp Met Leu
   545                 550                 555                 560
   att ggt gat cct ggg agg ttt cga caa ata ctc aca aat ctt atg ggt    1728
   Ile Gly Asp Pro Gly Arg Phe Arg Gln Ile Leu Thr Asn Leu Met Gly
                   565                 570                 575
   aat tcc att aag ttc act gag aaa gga cac atc ttt gta act gtt cat    1776
   Asn Ser Ile Lys Phe Thr Glu Lys Gly His Ile Phe Val Thr Val His
               580                 585                 590
   ttg gtg gat gag cta ttt gaa tct atc gat gga gag aca gca tca tct    1824
   Leu Val Asp Glu Leu Phe Glu Ser Ile Asp Gly Glu Thr Ala Ser Ser
           595                 600                 605
   ccg gaa agt aca ctg agt ggg ctt cca gtt gca gac cgg cag agg agc    1872
   Pro Glu Ser Thr Leu Ser Gly Leu Pro Val Ala Asp Arg Gln Arg Ser
       610                 615                 620
   tgg gaa aac ttt aaa gct ttc agc tcc aac ggg cat cgg agc ttt gaa    1920
   Trp Glu Asn Phe Lys Ala Phe Ser Ser Asn Gly His Arg Ser Phe Glu
   625                 630                 635                 640
   cca tct ccc cct gat ata aac cta atc gtc tca gtt gag gat act ggc    1968
   Pro Ser Pro Pro Asp Ile Asn Leu Ile Val Ser Val Glu Asp Thr Gly
                   645                 650                 655
   gta ggg atc cct gta gaa gcg cag tcc cgt att ttt acg cct ttc atg    2016
   Val Gly Ile Pro Val Glu Ala Gln Ser Arg Ile Phe Thr Pro Phe Met
               660                 665                 670
   caa gtc gga cca tcc ata tcc agg acg cat gga ggc aca gga att gga    2064
   Gln Val Gly Pro Ser Ile Ser Arg Thr His Gly Gly Thr Gly Ile Gly
           675                 680                 685
   ctt agc ata agc aaa tgt cta gtt gga ctg atg aag gga gaa att gga    2112
   Leu Ser Ile Ser Lys Cys Leu Val Gly Leu Met Lys Gly Glu Ile Gly
       690                 695                 700
   ttc tcg agt act ccc aag gtt ggg tcc aca ttc aca ttt act gct gta    2160
   Phe Ser Ser Thr Pro Lys Val Gly Ser Thr Phe Thr Phe Thr Ala Val
   705                 710                 715                 720
   ttt tcc aat ggg atg caa cca gct gaa aga aag aat gac aac aac cag    2208
   Phe Ser Asn Gly Met Gln Pro Ala Glu Arg Lys Asn Asp Asn Asn Gln
                   725                 730                 735
   ccc ata ttc tcg gaa ttc cgg ggc atg aaa gct gtg gtt gtg gac cat    2256
   Pro Ile Phe Ser Glu Phe Arg Gly Met Lys Ala Val Val Val Asp His
               740                 745                 750
   agg cct gca agg gca aaa gtc tcg tgg tac cat ttt cag cgt ctt gga    2304
   Arg Pro Ala Arg Ala Lys Val Ser Trp Tyr His Phe Gln Arg Leu Gly
           755                 760                 765
   att cga gtc gaa gta gtt cca cgt gtt gaa cag gct cta cat tat ctg    2352
   Ile Arg Val Glu Val Val Pro Arg Val Glu Gln Ala Leu His Tyr Leu
       770                 775                 780
   aag att ggt act acc act gtg aat atg ata ctc ata gag caa gaa ata    2400
   Lys Ile Gly Thr Thr Thr Val Asn Met Ile Leu Ile Glu Gln Glu Ile
   785                 790                 795                 800
   tgg aat agg gaa gca gat gat ttc att aaa aag cta cag aaa gac cct    2448
   Trp Asn Arg Glu Ala Asp Asp Phe Ile Lys Lys Leu Gln Lys Asp Pro
                   805                 810                 815
   ctt ttc ctt tct cct aag ttg att ttg tta gca aac tca gta gaa tcg    2496
   Leu Phe Leu Ser Pro Lys Leu Ile Leu Leu Ala Asn Ser Val Glu Ser
               820                 825                 830
   tca ata tca gag gct tta tgc acc ggt ata gat cct cca ata gtg ata     2544
   Ser Ile Ser Glu Ala Leu Cys Thr Gly Ile Asp Pro Pro Ile Val Ile
           835                 840                 845
   gtg aaa cca ttg agg gcg agt atg cta gca gca act ttg cag agg gga     2592
   Val Lys Pro Leu Arg Ala Ser Met Leu Ala Ala Thr Leu Gln Arg Gly
       850                 855                 860
   ttg ggt att gga atc aga gaa cca cct caa cac aag gga cct cct gct     2640
   Leu Gly Ile Gly Ile Arg Glu Pro Pro Gln His Lys Gly Pro Pro Ala
   865                 870                 875                 880
   ttg att ctc agg aat ctt ctc ctt ggt aga aaa att tta atc gtg gat     2688
   Leu Ile Leu Arg Asn Leu Leu Leu Gly Arg Lys Ile Leu Ile Val Asp
                   885                 890                 895
   gat aac aac gta aac ctc aga gtg gca gcg gga gct ctg aaa aag tac     2736
   Asp Asn Asn Val Asn Leu Arg Val Ala Ala Gly Ala Leu Lys Lys Tyr
               900                 905                 910
   gga gct gat gtg gtc tgc gct gag agt ggg ata aag gca atc tca ttg     2784
   Gly Ala Asp Val Val Cys Ala Glu Ser Gly Ile Lys Ala Ile Ser Leu
           915                 920                 925
   ctt aag cca cct cac gag ttt gat gct tgc ttc atg gac att cag atg     2832
   Leu Lys Pro Pro His Glu Phe Asp Ala Cys Phe Met Asp Ile Gln Met
       930                 935                 940
   cca gaa atg gat gga ttt gaa gct aca agg aga ata cga gat atg gaa     2880
   Pro Glu Met Asp Gly Phe Glu Ala Thr Arg Arg Ile Arg Asp Met Glu
   945                 950                 955                 960
   gag gag atg aac aag aga ata aag aat ggg gag gct ttg ata gta gag     2928
   Glu Glu Met Asn Lys Arg Ile Lys Asn Gly Glu Ala Leu Ile Val Glu
                   965                 970                 975
   aac ggt aac aaa aca agc tgg cat ctt ccg gta tta gca atg acg gca     2976
   Asn Gly Asn Lys Thr Ser Trp His Leu Pro Val Leu Ala Met Thr Ala
               980                 985                 990
   gat gtg atc caa gca acg cat gag gaa tgt ctg aag tgt gga atg gat     3024
   Asp Val Ile Gln Ala Thr His Glu Glu Cys Leu Lys Cys Gly Met Asp
           995                 1000                1005
   ggg tat gta tca aaa cca ttt gaa gca gag cag ctg tac agg gaa gtt     3072
   Gly Tyr Val Ser Lys Pro Phe Glu Ala Glu Gln Leu Tyr Arg Glu Val
       1010                1015                1020
   tct cgc ttt ttc aat tcg cct tca gat aca gaa tca taa                 3111
   Ser Arg Phe Phe Asn Ser Pro Ser Asp Thr Glu Ser
   1025                1030                1035
<210> 4
   <211> 1036
   <212> PRT
   <213> Arabidopsis thaliana
<400> 4
   Met Ser Leu Phe His Val Leu Gly Phe Gly Val Lys Ile Gly His Leu
    1               5                  10                  15
   Phe Trp Met Leu Cys Cys Trp Phe Val Ser Trp Phe Val Asp Asn Gly
                       20                  25                  30
   Ile Glu Asp Lys Ser Gly Leu Leu Val Gly Ser Val Gly Asp Leu Glu
                   35                  40                  45
   Lys Thr Lys Met Thr Thr Leu Lys Lys Lys Asn Lys Met Trp Phe Trp
               50                  55                  60
   Asn Lys Ile Ser Ser Ser Gly Leu Lys Ile Pro Ser Phe Ser Tyr Gln
    65                  70                  75                  80
   Phe Leu Gly Ser Val Lys Phe Asn Lys Ala Trp Trp Arg Lys Leu Val
                       85                  90                  95
   Val Val Trp Val Val Phe Trp Val Leu Val Ser Ile Trp Thr Phe Trp
                   100                 105                 110
   Tyr Phe Ser Ser Gln Ala Met Glu Lys Arg Lys Glu Thr Leu Ala Ser
               115                 120                 125
   Met Cys Asp Glu Arg Ala Arg Met Leu Gln Asp Gln Phe Asn Val Ser
           130                 135                 140
   Met Asn His Val Gln Ala Met Ser Ile Leu Ile Ser Thr Phe His His
   145                 150                 155                 160
   Gly Lys Ile Pro Ser Ala Ile Asp Gln Arg Thr Phe Ser Glu Tyr Thr
                   165                 170                 175
   Asp Arg Thr Ser Phe Glu Arg Pro Leu Thr Ser Gly Val Ala Tyr Ala
               180                 185                 190
   Met Arg Val Leu His Ser Glu Arg Glu Glu Phe Glu Arg Gln Gln Gly
           195                 200                 205
   Trp Thr Ile Arg Lys Met Tyr Ser Leu Glu Gln Asn Pro Val His Lys
       210                 215                 220
   Asp Asp Tyr Asp Leu Glu Ala Leu Glu Pro Ser Pro Val Gln Glu Glu
   225                 230                 235                 240
   Tyr Ala Pro Val Ile Phe Ala Gln Asp Thr Val Ser His Val Val Ser
                   245                 250                 255
   Leu Asp Met Leu Ser Gly Lys Glu Asp Arg Glu Asn Val Leu Arg Ala
               260                 265                 270
   Arg Ser Ser Gly Lys Gly Val Leu Thr Ala Pro Phe Pro Leu Ile Lys
           275                 280                 285
   Thr Asn Arg Leu Gly Val Ile Leu Thr Phe Ala Val Tyr Lys Arg Asp
       290                 295                 300
   Leu Pro Ser Asn Ala Thr Pro Lys Glu Arg Ile Glu Ala Thr Asn Gly
   305                 310                 315                 320
   Tyr Leu Gly Gly Val Phe Asp Ile Glu Ser Leu Val Glu Asn Leu Leu
                   325                 330                 335
   Gln Gln Leu Ala Ser Lys Gln Thr Ile Leu Val Asn Val Tyr Asp Ile
               340                 345                 350
   Thr Asn His Ser Gln Pro Ile Ser Met Tyr Gly Thr Asn Val Ser Ala
           355                 360                 365
   Asp Gly Leu Glu Arg Val Ser Pro Leu Ile Phe Gly Asp Pro Leu Arg
       370                 375                 380
   Lys His Glu Met Arg Cys Arg Phe Lys Gln Lys Pro Pro Trp Pro Val
   385                 390                 395                 400
   Leu Ser Met Val Thr Ser Phe Gly Ile Leu Val Ile Ala Leu Leu Val
                   405                 410                 415
   Ala His Ile Ile His Ala Thr Val Ser Arg Ile His Lys Val Glu Glu
               420                 425                 430
   Asp Cys Asp Lys Met Lys Gln Leu Lys Lys Lys Ala Glu Ala Ala Asp
           435                 440                 445
   Val Ala Lys Ser Gln Phe Leu Ala Thr Val Ser His Glu Ile Arg Thr
       450                 455                 460
   Pro Met Asn Gly Val Leu Gly Met Leu His Met Leu Met Asp Thr Glu
   465                 470                 475                 480
   Leu Asp Val Thr Gln Gln Asp Tyr Val Arg Thr Ala Gln Ala Ser Gly
                   485                 490                 495
   Lys Ala Leu Val Ser Leu Ile Asn Glu Val Leu Asp Gln Ala Lys Ile
               500                 505                 510
   Glu Ser Gly Lys Leu Glu Leu Glu Glu Val Arg Phe Asp Leu Arg Gly
           515                 520                 525
   Ile Leu Asp Asp Val Leu Ser Leu Phe Ser Ser Lys Ser Gln Gln Lys
       530                 535                 540
   Gly Val Glu Leu Ala Val Tyr Ile Ser Asp Arg Val Pro Asp Met Leu
   545                 550                 555                 560
   Ile Gly Asp Pro Gly Arg Phe Arg Gln Ile Leu Thr Asn Leu Met Gly
                   565                 570                 575
   Asn Ser Ile Lys Phe Thr Glu Lys Gly His Ile Phe Val Thr Val His
               580                 585                 590
   Leu Val Asp Glu Leu Phe Glu Ser Ile Asp Gly Glu Thr Ala Ser Ser
           595                 600                 605
   Pro Glu Ser Thr Leu Ser Gly Leu Pro Val Ala Asp Arg Gln Arg Ser
       610                 615                 620
   Trp Glu Asn Phe Lys Ala Phe Ser Ser Asn Gly His Arg Ser Phe Glu
   625                 630                 635                 640
   Pro Ser Pro Pro Asp Ile Asn Leu Ile Val Ser Val Glu Asp Thr Gly
                   645                 650                 655
   Val Gly Ile Pro Val Glu Ala Gln Ser Arg Ile Phe Thr Pro Phe Met
               660                 665                 670
   Gln Val Gly Pro Ser Ile Ser Arg Thr His Gly Gly Thr Gly Ile Gly
           675                 680                 685
   Leu Ser Ile Ser Lys Cys Leu Val Gly Leu Met Lys Gly Glu Ile Gly
       690                 695                 700
   Phe Ser Ser Thr Pro Lys Val Gly Ser Thr Phe Thr Phe Thr Ala Val
   705                 710                 715                 720
   Phe Ser Asn Gly Met Gln Pro Ala Glu Arg Lys Asn Asp Asn Asn Gln
                   725                 730                 735
   Pro Ile Phe Ser Glu Phe Arg Gly Met Lys Ala Val Val Val Asp His
               740                 745                 750
   Arg Pro Ala Arg Ala Lys Val Ser Trp Tyr His Phe Gln Arg Leu Gly
           755                 760                 765
   Ile Arg Val Glu Val Val Pro Arg Val Glu Gln Ala Leu His Tyr Leu
       770                 775                 780
   Lys Ile Gly Thr Thr Thr Val Asn Met Ile Leu Ile Glu Gln Glu Ile
   785                 790                 795                 800
   Trp Asn Arg Glu Ala Asp Asp Phe Ile Lys Lys Leu Gln Lys Asp Pro
                   805                 810                 815
   Leu Phe Leu Ser Pro Lys Leu Ile Leu Leu Ala Asn Ser Val Glu Ser
               820                 825                 830
   Ser Ile Ser Glu Ala Leu Cys Thr Gly Ile Asp Pro Pro Ile Val Ile
           835                 840                 845
   Val Lys Pro Leu Arg Ala Ser Met Leu Ala Ala Thr Leu Gln Arg Gly
       850                 855                 860
   Leu Gly Ile Gly Ile Arg Glu Pro Pro Gln His Lys Gly Pro Pro Ala
   865                 870                 875                 880
   Leu Ile Leu Arg Asn Leu Leu Leu Gly Arg Lys Ile Leu Ile Val Asp
                   885                 890                 895
   Asp Asn Asn Val Asn Leu Arg Val Ala Ala Gly Ala Leu Lys Lys Tyr
               900                 905                 910
   Gly Ala Asp Val Val Cys Ala Glu Ser Gly Ile Lys Ala Ile Ser Leu
           915                 920                 925
   Leu Lys Pro Pro His Glu Phe Asp Ala Cys Phe Met Asp Ile Gln Met
       930                 935                 940
   Pro Glu Met Asp Gly Phe Glu Ala Thr Arg Arg Ile Arg Asp Met Glu
   945                 950                 955                 960
   Glu Glu Met Asn Lys Arg Ile Lys Asn Gly Glu Ala Leu Ile Val Glu
                   965                 970                 975
   Asn Gly Asn Lys Thr Ser Trp His Leu Pro Val Leu Ala Met Thr Ala
               980                 985                 990
   Asp Val Ile Gln Ala Thr His Glu Glu Cys Leu Lys Cys Gly Met Asp
           995                 1000                1005
   Gly Tyr Val Ser Lys Pro Phe Glu Ala Glu Gln Leu Tyr Arg Glu Val
      1010                1015                1020
   Ser Arg Phe Phe Asn Ser Pro Ser Asp Thr Glu Ser
   1025 1030 1035
<210> 5
   <211> 3174
   <212> DNA
   <213> Arabidopsis thaliana
<220>
   <221> CDS
   <222> (1)..(3174)
<400> 5
   atg aac tgg gca ctc aac aat cat caa gaa gaa gaa gaa gag cca cga    48
   Met Asn Trp Ala Leu Asn Asn His Gln Glu Glu Glu Glu Glu Pro Arg
    1               5                  10                  15
   aga att gaa att tct gat tcc gag tca cta gaa aac ttg aaa agc agc    96
   Arg Ile Glu Ile Ser Asp Ser Glu Ser Leu Glu Asn Leu Lys Ser Ser
               20                  25                  30
   gat ttt tat caa ctg ggt ggt ggt ggt gct ctg aat tcg tca gaa aag   144
   Asp Phe Tyr Gln Leu Gly Gly Gly Gly Ala Leu Asn Ser Ser Glu Lys
           35                  40                  45
   ccg aga aag atc gat ttt tgg cgt tcg ggg ttg atg ggt ttt gcg aag   192
   Pro Arg Lys Ile Asp Phe Trp Arg Ser Gly Leu Met Gly Phe Ala Lys
       50                  55                  60
   atg cag cag cag caa cag ctt cag cat tca gtg gcg gtg aag atg aac   240
   Met Gln Gln Gln Gln Gln Leu Gln His Ser Val Ala Val Lys Met Asn
   65                  70                  75                  80
   aat aat aat aat aac gat cta atg ggt aat aaa aaa ggg tca act ttc   288
   Asn Asn Asn Asn Asn Asp Leu Met Gly Asn Lys Lys Gly Ser Thr Phe
                   85                  90                  95
   ata caa gaa cat cga gca ttg tta cca aaa gct ttg att ctg tgg atc   336
   Ile Gln Glu His Arg Ala Leu Leu Pro Lys Ala Leu Ile Leu Trp Ile
               100                 105                 110
   atc att gtt ggg ttt ata agc agt ggg att tat cag tgg atg gat gat   384
   Ile Ile Val Gly Phe Ile Ser Ser Gly Ile Tyr Gln Trp Met Asp Asp
           115                 120                 125
   gct aat aag att aga agg gaa gag gtt ttg gtc agc atg tgt gat caa   432
   Ala Asn Lys Ile Arg Arg Glu Glu Val Leu Val Ser Met Cys Asp Gln
       130                 135                 140
   aga gct aga atg ttg cag gat caa ttt agt gtt agt gtt aat cat gtt   480
   Arg Ala Arg Met Leu Gln Asp Gln Phe Ser Val Ser Val Asn His Val
   145                 150                 155                 160
   cat gct ttg gct att ctc gtc tcc act ttt cat tac cac aag aac cct   528
   His Ala Leu Ala Ile Leu Val Ser Thr Phe His Tyr His Lys Asn Pro
                   165                 170                 175
   tct gca att gat cag gag aca ttt gcg gag tac acg gca aga aca gca   576
   Ser Ala Ile Asp Gln Glu Thr Phe Ala Glu Tyr Thr Ala Arg Thr Ala
               180                 185                 190
   ttt gag aga ccg ttg cta agt gga gtg gct tat gct gaa aaa gtt gtg   624
   Phe Glu Arg Pro Leu Leu Ser Gly Val Ala Tyr Ala Glu Lys Val Val
           195                 200                 205
   aat ttt gag agg gag atg ttt gag cgg cag cac aat tgg gtt ata aag     672
   Asn Phe Glu Arg Glu Met Phe Glu Arg Gln His Asn Trp Val Ile Lys
       210                 215                 220
   aca atg gat aga gga gag cct tca ccg gtt agg gat gag tat gct cct     720
   Thr Met Asp Arg Gly Glu Pro Ser Pro Val Arg Asp Glu Tyr Ala Pro
   225                 230                 235                 240
   gtt ata ttc tct caa gat agt gtc tct tac ctt gag tca ctc gat atg     768
   Val Ile Phe Ser Gln Asp Ser Val Ser Tyr Leu Glu Ser Leu Asp Met
                   245                 250                 255
   atg tca ggc gag gag gat cgt gag aat att ttg cga gct aga gaa acc     816
   Met Ser Gly Glu Glu Asp Arg Glu Asn Ile Leu Arg Ala Arg Glu Thr
               260                 265                 270
   gga aaa gct gtc ttg act agc cct ttt agg ttg ttg gaa act cac cat     864
   Gly Lys Ala Val Leu Thr Ser Pro Phe Arg Leu Leu Glu Thr His His
           275                 280                 285
   ctc gga gtt gtg ttg aca ttc cct gtc tac aag tct tct ctt cct gaa     912
   Leu Gly Val Val Leu Thr Phe Pro Val Tyr Lys Ser Ser Leu Pro Glu
       290                 295                 300
   aat ccg act gtc gaa gag cgt att gca gcc act gca ggg tac ctt ggt     960
   Asn Pro Thr Val Glu Glu Arg Ile Ala Ala Thr Ala Gly Tyr Leu Gly
   305                 310                 315                 320
   ggt gcg ttt gat gtg gag tct cta gtc gag aat tta ctt ggt cag ctt    1008
   Gly Ala Phe Asp Val Glu Ser Leu Val Glu Asn Leu Leu Gly Gln Leu
                   325                 330                 335
   gct ggt aac caa gca ata gtt gtg cat gtg tat gat atc acc aat gca    1056
   Ala Gly Asn Gln Ala Ile Val Val His Val Tyr Asp Ile Thr Asn Ala
               340                 345                 350
   tca gat cca ctt gtc atg tat ggt aat caa gat gaa gaa gcc gac aga    1104
   Ser Asp Pro Leu Val Met Tyr Gly Asn Gln Asp Glu Glu Ala Asp Arg
           355                 360                 365
   tct ctc tct cat gag agc aag ctc gat ttt gga gac ccc ttc agg aaa    1152
   Ser Leu Ser His Glu Ser Lys Leu Asp Phe Gly Asp Pro Phe Arg Lys
       370                 375                 380
   cat aag atg ata tgc agg tac cac caa aag gca cca ata cca ttg aat    1200
   His Lys Met Ile Cys Arg Tyr His Gln Lys Ala Pro Ile Pro Leu Asn
   385                 390                 395                 400
   gtg ctc aca act gtg cca ttg ttc ttt gcg att ggt ttc ttg gtg ggt    1248
   Val Leu Thr Thr Val Pro Leu Phe Phe Ala Ile Gly Phe Leu Val Gly
                   405                 410                 415
   tat ata ctg tat ggt gca gct atg cac ata gta aaa gtc gaa gat gat    1296
   Tyr Ile Leu Tyr Gly Ala Ala Met His Ile Val Lys Val Glu Asp Asp
               420                 425                 430
   ttc cat gaa atg caa gag ctt aaa gtg cga gca gaa gct gct gat gtc    1344
   Phe His Glu Met Gln Glu Leu Lys Val Arg Ala Glu Ala Ala Asp Val
           435                 440                 445
   gct aaa tcg cag ttt ctt gct acc gtg tct cac gag atc agg aca cca    1392
   Ala Lys Ser Gln Phe Leu Ala Thr Val Ser His Glu Ile Arg Thr Pro
       450                 455                 460
   atg aat ggc att ctc gga atg ctt gct atg ctc cta gat aca gaa cta    1440
   Met Asn Gly Ile Leu Gly Met Leu Ala Met Leu Leu Asp Thr Glu Leu
   465                 470                 475                 480
   agc tcg aca cag aga gat tac gct caa acc gct caa gta tgt ggt aaa    1488
   Ser Ser Thr Gln Arg Asp Tyr Ala Gln Thr Ala Gln Val Cys Gly Lys
                   485                 490                 495
   gct ttg att gca ttg ata aat gag gtt ctt gat cgc gcc aag att gaa    1536
   Ala Leu Ile Ala Leu Ile Asn Glu Val Leu Asp Arg Ala Lys Ile Glu
               500                 505                 510
   gct gga aag ctg gag ttg gaa tca gta cca ttt gat atc cgt tca ata    1584
   Ala Gly Lys Leu Glu Leu Glu Ser Val Pro Phe Asp Ile Arg Ser Ile
           515                 520                 525
   ttg gat gat gtc ctt tct cta ttc tct gag gag tca agg aac aaa ggc    1632
   Leu Asp Asp Val Leu Ser Leu Phe Ser Glu Glu Ser Arg Asn Lys Gly
       530                 535                 540
   att gag ctc gcg gtt ttc gtt tca gac aaa gta cca gag ata gtc aaa    1680
   Ile Glu Leu Ala Val Phe Val Ser Asp Lys Val Pro Glu Ile Val Lys
   545                 550                 555                 560
   gga gat tca ggg aga ttt aga cag ata atc ata aac ctt gtt gga aat    1728
   Gly Asp Ser Gly Arg Phe Arg Gln Ile Ile Ile Asn Leu Val Gly Asn
                   565                 570                 575
   tcg gtt aaa ttc aca gag aaa gga cat atc ttt gtt aaa gtc cat ctt    1776
   Ser Val Lys Phe Thr Glu Lys Gly His Ile Phe Val Lys Val His Leu
               580                 585                 590
   gcg gaa caa tca aaa gat gaa tct gaa ccg aaa aat gca ttg aat ggt    1824
   Ala Glu Gln Ser Lys Asp Glu Ser Glu Pro Lys Asn Ala Leu Asn Gly
           595                 600                 605
   gga gtg tct gaa gaa atg atc gtt gtt tcc aaa cag tca agt tac aac    1872
   Gly Val Ser Glu Glu Met Ile Val Val Ser Lys Gln Ser Ser Tyr Asn
       610                 615                 620
   aca ttg agc ggt tac gaa gct gct gat ggt cgg aat agc tgg gat tca    1920
   Thr Leu Ser Gly Tyr Glu Ala Ala Asp Gly Arg Asn Ser Trp Asp Ser
   625                 630                 635                 640
   ttc aag cat ttg gtc tct gag gag cag tca tta tcg gag ttt gat att    1968
   Phe Lys His Leu Val Ser Glu Glu Gln Ser Leu Ser Glu Phe Asp Ile
                   645                 650                 655
   tct agc aat gtt agg ctt atg gtt tca atc gaa gac acg ggt att gga    2016
   Ser Ser Asn Val Arg Leu Met Val Ser Ile Glu Asp Thr Gly Ile Gly
               660                 665                 670
   atc cct tta gtt gca caa ggc cgt gtg ttt atg ccg ttt atg caa gca    2064
   Ile Pro Leu Val Ala Gln Gly Arg Val Phe Met Pro Phe Met Gln Ala
           675                 680                 685
   gat agc tcg act tca aga aac tat gga ggt act ggt att ggt ttg agt    2112
   Asp Ser Ser Thr Ser Arg Asn Tyr Gly Gly Thr Gly Ile Gly Leu Ser
       690                 695                 700
   ata agc aag tgt ctt gtt gaa ctt atg cgt ggt cag ata aat ttc ata    2160
   Ile Ser Lys Cys Leu Val Glu Leu Met Arg Gly Gln Ile Asn Phe Ile
   705                 710                 715                 720
   agc cgg cct cat att gga agc acg ttc tgg ttc acg gct gtt tta gag    2208
   Ser Arg Pro His Ile Gly Ser Thr Phe Trp Phe Thr Ala Val Leu Glu
                   725                 730                 735
   aaa tgc gat aaa tgc agt gcg att aac cat atg aag aaa cct aat gtg    2256
   Lys Cys Asp Lys Cys Ser Ala Ile Asn His Met Lys Lys Pro Asn Val
               740                 745                 750
   gaa cac ttg cct tct act ttt aaa gga atg aaa gct ata gtt gtt gat    2304
   Glu His Leu Pro Ser Thr Phe Lys Gly Met Lys Ala Ile Val Val Asp
           755                 760                 765
   gct aag cct gtt aga gct gct gtg act aga tac cat atg aaa aga ctc    2352
   Ala Lys Pro Val Arg Ala Ala Val Thr Arg Tyr His Met Lys Arg Leu
       770                 775                 780
   gga atc aat gtt gat gtc gtg aca agt ctc aaa acc gct gtt gtt gca    2400
   Gly Ile Asn Val Asp Val Val Thr Ser Leu Lys Thr Ala Val Val Ala
   785                 790                 795                 800
   gct gct gcg ttt gaa aga aac ggt tct cct ctc cca aca aaa ccg caa    2448
   Ala Ala Ala Phe Glu Arg Asn Gly Ser Pro Leu Pro Thr Lys Pro Gln
                   805                 810                 815
   ctt gat atg atc tta gta gag aaa gat tca tgg att tca act gaa gat    2496
   Leu Asp Met Ile Leu Val Glu Lys Asp Ser Trp Ile Ser Thr Glu Asp
               820                 825                 830
   aat gac tca gag att cgt tta ttg aat tca aga acc aac gga aac gtt    2544
   Asn Asp Ser Glu Ile Arg Leu Leu Asn Ser Arg Thr Asn Gly Asn Val
           835                 840                 845
   cat cac aag tct ccg aaa cta gct cta ttc gca aca aac atc aca aat    2592
   His His Lys Ser Pro Lys Leu Ala Leu Phe Ala Thr Asn Ile Thr Asn
       850                 855                 860
   tcg gag ttc gac aga gct aaa tcc gca gga ttt gca gat acg gta ata    2640
   Ser Glu Phe Asp Arg Ala Lys Ser Ala Gly Phe Ala Asp Thr Val Ile
   865                 870                 875                 880
   atg aaa ccg tta aga gca agc atg att ggg gcg tgt ctg caa caa gtt    2688
   Met Lys Pro Leu Arg Ala Ser Met Ile Gly Ala Cys Leu Gln Gln Val
                   885                 890                 895
   ctc gag ctg aga aaa aca aga caa caa cat cca gaa gga tca tca ccc    2736
   Leu Glu Leu Arg Lys Thr Arg Gln Gln His Pro Glu Gly Ser Ser Pro
               900                 905                 910
   gca act ctc aag agc ttg ctt aca ggg aag aag att ctt gtg gtt gat    2784
   Ala Thr Leu Lys Ser Leu Leu Thr Gly Lys Lys Ile Leu Val Val Asp
           915                 920                 925
   gat aat ata gtt aac agg aga gta gct gca gga gct ctc aag aaa ttt    2832
   Asp Asn Ile Val Asn Arg Arg Val Ala Ala Gly Ala Leu Lys Lys Phe
       930                 935                 940
   gga gca gaa gtg gtt tgt gca gag agt ggt caa gtt gct ttg ggt ttg    2880
   Gly Ala Glu Val Val Cys Ala Glu Ser Gly Gln Val Ala Leu Gly Leu
   945                 950                 955                 960
   ctt cag att cca cac act ttc gat gct tgc ttc atg gat att caa atg     2928
   Leu Gln Ile Pro His Thr Phe Asp Ala Cys Phe Met Asp Ile Gln Met
                   965                 970                 975
   cca cag atg gac gga ttt gaa gca act cgt cag ata aga atg atg gag     2976
   Pro Gln Met Asp Gly Phe Glu Ala Thr Arg Gln Ile Arg Met Met Glu
               980                 985                 990
   aag gaa gct aaa gag aag acg aat ctc gaa tgg cat tta ccg att cta     3024
   Lys Glu Ala Lys Glu Lys Thr Asn Leu Glu Trp His Leu Pro Ile Leu
           995                 1000                1005
   gcg atg act gcg gat gtg ata cac gcg acc tac gag gaa tgt ctg aaa     3072
   Ala Met Thr Ala Asp Val Ile His Ala Thr Tyr Glu Glu Cys Leu Lys
       1010                1015                1020
   agt ggg atg gat ggt tac gtc tcc aaa cct ttt gaa gaa gag aat ctc     3120
   Ser Gly Met Asp Gly Tyr Val Ser Lys Pro Phe Glu Glu Glu Asn Leu
   1025                1030                1035                1040
   tat aaa tcc gtt gcc aaa tca ttc aaa cct aat cct atc tca cct tcg     3168
   Tyr Lys Ser Val Ala Lys Ser Phe Lys Pro Asn Pro Ile Ser Pro Ser
                   1045                1050                1055
   tcg taa                                                             3174
   Ser
<210> 6
   <211> 1057
   <212> PRT
   <213> Arabidopsis thaliana
<400> 6
   Met Asn Trp Ala Leu Asn Asn His Gln Glu Glu Glu Glu Glu Pro Arg
    1               5                  10                  15
   Arg Ile Glu Ile Ser Asp Ser Glu Ser Leu Glu Asn Leu Lys Ser Ser
               20                  25                  30
   Asp Phe Tyr Gln Leu Gly Gly Gly Gly Ala Leu Asn Ser Ser Glu Lys
           35                  40                  45
   Pro Arg Lys Ile Asp Phe Trp Arg Ser Gly Leu Met Gly Phe Ala Lys
       50                  55                  60
   Met Gln Gln Gln Gln Gln Leu Gln His Ser Val Ala Val Lys Met Asn
   65                  70                  75                  80
   Asn Asn Asn Asn Asn Asp Leu Met Gly Asn Lys Lys Gly Ser Thr Phe
                   85                  90                  95
   Ile Gln Glu His Arg Ala Leu Leu Pro Lys Ala Leu Ile Leu Trp Ile
               100                 105                 110
   Ile Ile Val Gly Phe Ile Ser Ser Gly Ile Tyr Gln Trp Met Asp Asp
           115                 120                 125
   Ala Asn Lys Ile Arg Arg Glu Glu Val Leu Val Ser Met Cys Asp Gln
       130                 135                 140
   Arg Ala Arg Met Leu Gln Asp Gln Phe Ser Val Ser Val Asn His Val
   145                 150                 155                 160
   His Ala Leu Ala Ile Leu Val Ser Thr Phe His Tyr His Lys Asn Pro
                   165                 170                 175
   Ser Ala Ile Asp Gln Glu Thr Phe Ala Glu Tyr Thr Ala Arg Thr Ala
               180                 185                 190
   Phe Glu Arg Pro Leu Leu Ser Gly Val Ala Tyr Ala Glu Lys Val Val
           195                 200                 205
   Asn Phe Glu Arg Glu Met Phe Glu Arg Gln His Asn Trp Val Ile Lys
       210                 215                 220
   Thr Met Asp Arg Gly Glu Pro Ser Pro Val Arg Asp Glu Tyr Ala Pro
   225                 230                 235                 240
   Val Ile Phe Ser Gln Asp Ser Val Ser Tyr Leu Glu Ser Leu Asp Met
                   245                 250                 255
   Met Ser Gly Glu Glu Asp Arg Glu Asn Ile Leu Arg Ala Arg Glu Thr
               260                 265                 270
   Gly Lys Ala Val Leu Thr Ser Pro Phe Arg Leu Leu Glu Thr His His
           275                 280                 285
   Leu Gly Val Val Leu Thr Phe Pro Val Tyr Lys Ser Ser Leu Pro Glu
       290                 295                 300
   Asn Pro Thr Val Glu Glu Arg Ile Ala Ala Thr Ala Gly Tyr Leu Gly
   305                 310                 315                 320
   Gly Ala Phe Asp Val Glu Ser Leu Val Glu Asn Leu Leu Gly Gln Leu
                   325                 330                 335
   Ala Gly Asn Gln Ala Ile Val Val His Val Tyr Asp Ile Thr Asn Ala
               340                 345                 350
   Ser Asp Pro Leu Val Met Tyr Gly Asn Gln Asp Glu Glu Ala Asp Arg
           355                 360                 365
   Ser Leu Ser His Glu Ser Lys Leu Asp Phe Gly Asp Pro Phe Arg Lys
       370                 375                 380
   His Lys Met Ile Cys Arg Tyr His Gln Lys Ala Pro Ile Pro Leu Asn
   385                 390                 395                 400
   Val Leu Thr Thr Val Pro Leu Phe Phe Ala Ile Gly Phe Leu Val Gly
                   405                 410                 415
   Tyr Ile Leu Tyr Gly Ala Ala Met His Ile Val Lys Val Glu Asp Asp
               420                 425                 430
   Phe His Glu Met Gln Glu Leu Lys Val Arg Ala Glu Ala Ala Asp Val
           435                 440                 445
   Ala Lys Ser Gln Phe Leu Ala Thr Val Ser His Glu Ile Arg Thr Pro
       450                 455                 460
   Met Asn Gly Ile Leu Gly Met Leu Ala Met Leu Leu Asp Thr Glu Leu
   465                 470                 475                 480
   Ser Ser Thr Gln Arg Asp Tyr Ala Gln Thr Ala Gln Val Cys Gly Lys
                   485                 490                 495
   Ala Leu Ile Ala Leu Ile Asn Glu Val Leu Asp Arg Ala Lys Ile Glu
               500                 505                 510
   Ala Gly Lys Leu Glu Leu Glu Ser Val Pro Phe Asp Ile Arg Ser Ile
           515                 520                 525
   Leu Asp Asp Val Leu Ser Leu Phe Ser Glu Glu Ser Arg Asn Lys Gly
       530                 535                 540
   Ile Glu Leu Ala Val Phe Val Ser Asp Lys Val Pro Glu Ile Val Lys
   545                 550                 555                 560
   Gly Asp Ser Gly Arg Phe Arg Gln Ile Ile Ile Asn Leu Val Gly Asn
                   565                 570                 575
   Ser Val Lys Phe Thr Glu Lys Gly His Ile Phe Val Lys Val His Leu
               580                 585                 590
   Ala Glu Gln Ser Lys Asp Glu Ser Glu Pro Lys Asn Ala Leu Asn Gly
           595                 600                 605
   Gly Val Ser Glu Glu Met Ile Val Val Ser Lys Gln Ser Ser Tyr Asn
       610                 615                 620
   Thr Leu Ser Gly Tyr Glu Ala Ala Asp Gly Arg Asn Ser Trp Asp Ser
   625                 630                 635                 640
   Phe Lys His Leu Val Ser Glu Glu Gln Ser Leu Ser Glu Phe Asp Ile
                   645                 650                 655
   Ser Ser Asn Val Arg Leu Met Val Ser Ile Glu Asp Thr Gly Ile Gly
               660                 665                 670
   Ile Pro Leu Val Ala Gln Gly Arg Val Phe Met Pro Phe Met Gln Ala
           675                 680                 685
   Asp Ser Ser Thr Ser Arg Asn Tyr Gly Gly Thr Gly Ile Gly Leu Ser
       690                 695                 700
   Ile Ser Lys Cys Leu Val Glu Leu Met Arg Gly Gln Ile Asn Phe Ile
   705                 710                 715                 720
   Ser Arg Pro His Ile Gly Ser Thr Phe Trp Phe Thr Ala Val Leu Glu
                   725                 730                 735
   Lys Cys Asp Lys Cys Ser Ala Ile Asn His Met Lys Lys Pro Asn Val
               740                 745                 750
   Glu His Leu Pro Ser Thr Phe Lys Gly Met Lys Ala Ile Val Val Asp
           755                 760                 765
   Ala Lys Pro Val Arg Ala Ala Val Thr Arg Tyr His Met Lys Arg Leu
       770                 775                 780
   Gly Ile Asn Val Asp Val Val Thr Ser Leu Lys Thr Ala Val Val Ala
   785                 790                 795                 800
   Ala Ala Ala Phe Glu Arg Asn Gly Ser Pro Leu Pro Thr Lys Pro Gln
                   805                 810                 815
   Leu Asp Met Ile Leu Val Glu Lys Asp Ser Trp Ile Ser Thr Glu Asp
               820                 825                 830
   Asn Asp Ser Glu Ile Arg Leu Leu Asn Ser Arg Thr Asn Gly Asn Val
           835                 840                 845
   His His Lys Ser Pro Lys Leu Ala Leu Phe Ala Thr Asn Ile Thr Asn
       850                 855                 860
   Ser Glu Phe Asp Arg Ala Lys Ser Ala Gly Phe Ala Asp Thr Val Ile
   865                 870                 875                 880
   Met Lys Pro Leu Arg Ala Ser Met Ile Gly Ala Cys Leu Gln Gln Val
                   885                 890                 895
   Leu Glu Leu Arg Lys Thr Arg Gln Gln His Pro Glu Gly Ser Ser Pro
               900                 905                 910
   Ala Thr Leu Lys Ser Leu Leu Thr Gly Lys Lys Ile Leu Val Val Asp
           915                 920                 925
   Asp Asn Ile Val Asn Arg Arg Val Ala Ala Gly Ala Leu Lys Lys Phe
       930                 935                 940
   Gly Ala Glu Val Val Cys Ala Glu Ser Gly Gln Val Ala Leu Gly Leu
   945                 950                 955                 960
   Leu Gln Ile Pro His Thr Phe Asp Ala Cys Phe Met Asp Ile Gln Met
                   965                 970                 975
   Pro Gln Met Asp Gly Phe Glu Ala Thr Arg Gln Ile Arg Met Met Glu
               980                 985                 990
   Lys Glu Ala Lys Glu Lys Thr Asn Leu Glu Trp His Leu Pro Ile Leu
           995                 1000                1005
   Ala Met Thr Ala Asp Val Ile His Ala Thr Tyr Glu Glu Cys Leu Lys
       1010                1015                1020
   Ser Gly Met Asp Gly Tyr Val Ser Lys Pro Phe Glu Glu Glu Asn Leu
   1025                1030                1035                1040
   Tyr Lys Ser Val Ala Lys Ser Phe Lys Pro Asn Pro Ile Ser Pro Ser
                   1045                1050                1055
   Ser
<210> 7
   <211> 125
   <212> PRT
   <213> Saccharomyces cerevisiae
<400> 7
   Glu Thr Ser Val Lys Ile Leu Val Val Glu Asp Asn His Val Asn Gln
   1 5 10 15
   Glu Val Ile Lys Arg Met Leu Asn Leu Glu Gly Ile Glu Asn Ile Glu
   20 25 30
   Leu Ala Cys Asp Gly Gln Glu Ala Phe Asp Lys Val Lys Glu Leu Thr
   35 40 45
   Ser Lys Gly Glu Asn Tyr Asn Met Ile Phe Met Asp Val Gln Met Pro
   50 55 60
   Lys Val Asp Gly Leu Leu Ser Thr Lys Met Ile Arg Arg Asp Leu Gly
   65 70 75 80
   Tyr Thr Ser Pro Ile Val Ala Leu Thr Ala Phe Ala Asp Asp Ser Asn
   85 90 95
   Ile Lys Glu Cys Leu Glu Ser Gly Met Asn Gly Phe Leu Ser Lys Pro
   100 105 110
   Ile Lys Arg Pro Lys Leu Lys Thr Ile Leu Thr Glu Phe
   115 120 125
<210> 8
   <211> 118
   <212> PRT
   <213> Escherichia coli
<400> 1
<210> 9
   <211> 32
   <212> DNA
   <213> Artificial Sequence
<220>
   <223> Description of Artificial Sequence:Designed oligonucleotide primer for PCR
<400> 9
   tccccgcgga aaatgttctt acggttaggt ag 32
<210> 10
   <211> 33
   <212> DNA
   <213> Artificial Sequence
<220>
   <223> Description of Artificial Sequence:Designed oligonucleotide primer for PCR
<400> 10
   tcggtcgact tatgattctg tatctgaagg cga 33
<210> 11
   <211> 24
   <212> DNA
   <213> Artificial Sequence
<220>
   <223> Description of Artificial Sequence:Designed oligonucleotide primer for PCR
<400> 11
   tcagatatga actgggcact caac 24
<210> 12
   <211> 24
   <212> DNA
   <213> Artificial Sequence
<220>
   <223> Description of Artificial Sequence:Designed oligonucleotide primer for PCR
<400> 12
   ctcaatgctt ttgttccttg actc 24
<210> 13
   <211> 31
   <212> DNA
   <213> Artificial Sequence
<220>
   <223> Description of Artificial Sequence:Designed oligonucleotide primer for PCR
<400> 13
   accatgaact gggcactcaa caatcatcaa g 31
<210> 14
   <211> 30
   <212> DNA
   <213> Artificial Sequence
<220>
   <223> Description of Artificial Sequence:Designed oligonucleotide primer for PCR
<400> 14
   ggattacgac gaaggtgaga taggattagg 30
<210> 15
   <211> 32
   <212> DNA
   <213> Artificial Sequence
<220>
   <223> Description of Artificial Sequence:Designed oligonucleotide primer for PCR
<400> 15
   gatcccagct agctagggcc ctaccgcggg ga 32
<210> 16
   <211> 32
   <212> DNA
   <213> Artificial Sequence
<220>
   <223> Description of Artificial Sequence:Designed oligonucleotide primer for PCR
<400> 16
   tccccgcgga aaatgttctt acggttaggt ag 32
<210> 17
   <211> 33
   <212> DNA
   <213> Artificial Sequence
<220>
   <223> Description of Artificial Sequence:Designed oligonucleotide primer for PCR
<400> 17
   tcggtcgact tatgattctg tatctgaagg cga 33
<210> 18
   <211> 32
   <212> DNA
   <213> Artificial Sequence
<220>
   <223> Description of Artificial Sequence:Designed oligonucleotide primer for PCR
<400> 18
   ctagtccccg cggtagggcc ctagctagct gg 32
<210> 19
   <211> 31
   <212> DNA
   <213> Artificial Sequence
<220>
   <223> Description of Artificial Sequence:Designed oligonucleotide primer for PCR
<400> 19
   tccccgcgga aaatgtctat aacttgtgag c 31
<210> 20
   <211> 33
   <212> DNA
   <213> Artificial Sequence
<220>
   <223> Description of Artificial Sequence:Designed oligonucleotide primer for PCR
<400> 20
   ctagctagct taacaaggtt caaagaatct tgc 33
<210> 21
   <211> 33
   <212> DNA
   <213> Artificial Sequence
<220>
   <223> Description of Artificial Sequence:Designed oligonucleotide primer for PCR
<400> 21
   tccccgcgga aaatgaaagc acgaggtgag agg 33
<210> 22
   <211> 32
   <212> DNA
   <213> Artificial Sequence
<220>
   <223> Description of Artificial Sequence:Designed oligonucleotide primer for PCR
<400> 22
   ctagctagct taacaaggtt caaagaattt gc 32